(19) Europäisches Patentamt European Patent Office Office européen des brevets

(11) **EP 4 590 235 B1**

(12) **EUROPEAN PATENT SPECIFICATION**

(45) Date of publication and mention of the grant of the patent:
**22.07.2026 Bulletin 2026/30**

(21) Application number: **23809314.0**

(22) Date of filing: **11.11.2023**

(51) International Patent Classification (IPC):
**A61F 2/06** (2013.01) **A61F 2/07** (2013.01)
**A61F 2/856** (2013.01) **A61F 2/915** (2013.01)

(52) Cooperative Patent Classification (CPC):
**A61F 2/06; A61F 2/856;** A61F 2/07; A61F 2/915;
A61F 2002/061; A61F 2002/068; A61F 2002/9528;
A61F 2210/0076; A61F 2220/0008;
A61F 2220/0075; A61F 2230/001

(86) International application number:
**PCT/IB2023/061404**

(87) International publication number:
**WO 2024/105526 (23.05.2024 Gazette 2024/21)**

(54) **OPTIMIZED DEVICES FOR MODIFYING FLOW IN BODY LUMENS**

OPTIMIERTE VORRICHTUNGEN ZUR MODIFIZIERUNG DES FLUSSES IN KÖRPERLUMEN

DISPOSITIFS OPTIMISÉS POUR MODIFIER UN ÉCOULEMENT DANS DES LUMIÈRES CORPORELLES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.11.2022 US 202263383884 P**
**20.03.2023 US 202363491235 P**

(43) Date of publication of application:
**30.07.2025 Bulletin 2025/31**

(73) Proprietor: **Nephronyx Ltd.**
**7178115 Modiin (IL)**

(72) Inventors:
• **ROTMAN, Oren Moshe**
**5846003 Holon (IL)**
• **KARAVANY, Sagy**
**8533000 Kibbutz Dvir (IL)**
• **YACOBY, Menashe**
**6082110 Shoham (IL)**

(74) Representative: **Vossius & Partner**
**Patentanwälte Rechtsanwälte mbB**
**Siebertstrasse 3**
**81675 München (DE)**

(56) References cited:
WO-A1-2021/240411      WO-A2-2021/226014
US-A1- 2022 287 831

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

CROSS-REFERENCE TO RELATED APPLICATIONS

This application claims the benefit of priority to U.S. Provisional Patent Application No. 63/491,235, filed March 20, 2023, and U.S. Provisional Patent Application No. 63/383,884, filed on November 15, 2022.

FIELD OF THE INVENTION

The present invention relates generally to acute and chronic devices for modifying flow in body lumens, such as optimized devices for creating pressure differences and/or entrainment of fluid at lumens that branch off from other lumens for enhancing fluid flow to treat different disorders or diseases.

BACKGROUND OF THE INVENTION

Heart failure is the physiological state in which cardiac output is insufficient to meet the needs of the body and the lungs. Patients suffering from any of a number of forms of heart failure are prone to increased fluid in the body. Congestive heart failure (CHF) occurs when cardiac output is relatively low and the body becomes congested with fluid. There are many possible underlying causes of CHF, including myocardial infarction, coronary artery disease, valvular disease, and myocarditis. Chronic heart failure is associated with neurohormonal activation and alterations in autonomic control. Although these compensatory neurohormonal mechanisms provide valuable support for the heart under normal physiological circumstances, they also have a fundamental role in the development and subsequent progression of CHF. For example, one of the body's main compensatory mechanisms for reduced blood flow in CHF is to increase the amount of salt and water retained by the kidneys. Retaining salt and water, instead of excreting it into the urine, increases the volume of blood in the bloodstream and helps to maintain blood pressure. However, the larger volume of blood also stretches the heart muscle, enlarging the heart chambers, particularly the ventricles. At a certain amount of stretching, the heart's contractions become weakened, and the heart failure worsens. Another compensatory mechanism is vasoconstriction of the arterial system. This mechanism, like salt and water retention, raises the blood pressure to help maintain adequate perfusion.

Glomerular filtration rate (GFR), the rate at which the kidney filters blood, is commonly used to quantify kidney function and, consequently, the extent of kidney disease in a patient. Individuals with normal kidney function exhibit a GFR of at least 90 mL/min with no evidence of kidney damage. The progression of kidney disease is indicated by declining GFR, wherein a GFR below 15 mL/min generally indicates that the patient has end stage renal disease (ESRD), which is the complete failure of the kidney to remove wastes or concentrate urine.

In addition to increases in total body salt and water, it has also been found that altered capacitance of the splanchnic venous vessels change the blood volume distribution. Decreased venous capacitance can lead to shifts of fluid from the venous reservoir into the effective circulatory volume/splanchnic circulation, thus increasing filling pressures. This could result in clinical heart congestion.

Cardiovascular problems, such as but not limited to, inadequate blood flow or chronic hypertension, may lead to fluid retention in the kidneys, chronic kidney disease, lowered GFR, renal failure or even ESRD. For example, hypertension is considered the second most prevalent cause for kidney failure (after diabetes). It has been estimated that hypertension causes nephrotic damage and lowers GFR.

Transjugular intrahepatic portosystemic shunt (TIPS or TIPSS) is an artificial channel within the liver that establishes communication between the inflow portal vein and the outflow hepatic vein. Generally, under imaging guidance, a small metal stent is placed to keep the channel open and allow the channel to bring blood draining from the bowel back to the heart while avoiding the liver. TIPS may be used to treat conditions such as portal hypertension (often due to liver cirrhosis) which frequently leads to intestinal bleeding, life-threatening esophageal bleeding (esophageal varices), and the buildup of fluid within the abdomen (ascites), and has shown promise for treating hepatorenal syndrome. A drawback of TIPS is that blood meant to be filtered by the liver bypasses the liver via the artificial channel, which may cause complications.

Therefore, it would be desirable to provide acute and/or chronic apparatus and methods to improve blood flow to prevent disease, improve body functionality, and/or treat conditions that would benefit from modified body fluid flow. For example, it would be desirable to treat heart failure, treat hypertension, prevent kidney disease, improve kidney functionality, restore normal values of splanchnic circulation, improve liver functionality, enhance or replace TIPS, and/or prevent blood clots from flowing through vasculature to sensitive portions of the body, such as the brain, in order to prevent strokes.

It would further be desirable to provide a flow modulator device with efficacy across a wide range of blood flow.

SUMMARY OF THE INVENTION

The present invention is defined in appended claim 1. Preferential embodiments of the invention are recited in the dependent claims. The present invention seeks to provide acute and chronic devices for altering flow in body lumens. For example, devices are provided for creating pressure differences and/or fluid entrainment at lumens that branch off from other lumens for enhancing or modifying fluid flow to treat different disorders or diseases. For positioning, the device may be acutely or chronically implanted within the body lumen.

The devices of the present invention have many applications. For example, the device may be used to reduce pressure and improve flow, thereby improving flow in stenotic body lumens. It also may be used in the aortic arch to reduce peak systolic pressure in the brain or divert emboli to other portions of the body (e.g., the legs) and thereby reduce the risk of stroke. The device further may be installed in a bifurcation (e.g., in the brachiocephalic vessels) to reduce peak pressure gradients or to divert emboli with very little energy loss.

The devices of the present invention have particular application in treating blood flow to and from the kidneys. In accordance with one embodiment, the device is configured to be installed near one of the renal arteries or in the inferior vena cava near the branch off to the renal veins or in one of the renal veins. When installed in the inferior vena cava or in the renal vein, the device can create (due to the Bernoulli effect or other factors) a region in the inferior vena cava or in the renal vein which has increased blood velocity and reduced pressure. In this manner, blood may be drawn from the kidneys to the renal veins and then to the inferior vena cava, thereby improving kidney functionality and reducing necrotic damage to the kidneys.

When installed in or near the renal vein, the devices of the present invention may improve renal function by improving net filtration pressure, which is glomerular capillary blood pressure - (plasma-colloid osmotic pressure + Bowman's capsule hydrostatic pressure), e.g., 55 mm Hg - (30 mm Hg + 15 mm Hg) = 10 mm Hg. The devices of the present invention thus provide an improvement over existing therapies, such as diuretics (although the invention can be used in addition to diuretics), angiotensin-converting enzyme inhibitors (ACEIs), and angiotensin receptor blockers (ARBs), which can have deleterious effects on kidney function. When used in conjunction with current modes of treatment such as diuretics, the devices of the present invention are expected to improve the response for diuretics and reduce the dosage needed to obtain therapeutic benefit of such previously known therapies, without the disadvantages of these existing therapies.

The devices of the present invention may be used to divert flow from the kidneys to the inferior vena cava with little energy loss. For example, with a small energy loss due to pressure drop and other fluid factors, a significantly greater increase in blood flow may be achieved. This diversion of flow from the kidneys with little energy loss to increase blood flow is expected to treat conditions such as heart failure and/or hypertension.

It is noted that there is a significant difference between use of an upstream nozzle with no downstream flow decelerator, such as a diffuser. If only an upstream nozzle is placed in the flow path, there is significant energy loss downstream of the nozzle due to the sudden expansion of flow. However, by using a downstream flow decelerator, such as a diffuser, the energy loss is significantly reduced. This leads to another advantage: since the energy loss is significantly reduced, the additional flow that flows through the entrainment region is efficiently added to the flow from the upstream flow accelerator.

In addition, the present invention is expected to provide optimal structure for an upstream flow accelerator when used together with a downstream flow decelerator. For example, the length of the entrainment region between the upstream flow accelerator and the i downstream flow decelerator should be less than a predetermined length to reduce pressure at the entrainment region between the outlet and the inlet.

When installed in the renal artery, the device can reduce pressure applied to the kidneys. Without being limited by any theory, high blood pressure can cause damage to the blood vessels and filters in the kidney, making removal of waste from the body difficult. By reducing the pressure in the renal artery, the filtration rate improves. Although there may be a reduction in the perfusion pressure, the filtration rate will increase because the overall kidney function is more efficient.

It is noted that the fluid flow modulator of the present invention may modulate fluid flow without any input from an external energy source, such as a fan, motor, and the like and without any moving parts. The structure of the device of the invention transfers energy from one lumen flow to another different lumen flow with minimal flow energy losses. Some of the energy loss (in the form of pressure loss and/or reduced volume) may beneficially reduce heart preload (i.e., the filling pressure). For example, in healthy patients, the Frank-Starling mechanism dictates that increases in preload will increase cardiac output; however, in the setting of impaired contractility (as is the case with many chronic and acute heart failure patients), this relation is reversed such that preload increases can cause a reduction in cardiac output. Thus, it is desirable to reduce preload in heart failure patients in order to improve cardiac function. It is especially desirable to reduce preload in heart failure patients with reduced renal blood flow as reduced as reduced renal blood flow leads to fluid accumulation which increases both filling pressure and renal congestion.

In accordance with the present invention, a flow modulator device for altering fluid flow through a body lumen coupled to a branch lumen is provided. The flow modulator includes an upstream component, a downstream component, and an entrainment region between the inlet of the upstream component and the exit of the downstream component. The upstream component has an inlet, an outlet, and a cross-sectional flow area that converges from the inlet towards the outlet to form a nozzle. The downstream component has an entry, an exit, and a cross-sectional flow area that diverges from the entry towards the exit to form a diffuser. In addition, the downstream component includes a first diverging portion and a second diverging portion downstream from the first diverging portion, such that the first diverging portion's average angle of divergence is greater than the second diverging portion's average angle of divergence. The entrainment region includes one or more openings extending across at least a portion of both the first and second diverging portions of the downstream component. Accordingly, the flow modulator device is configured to be positioned within the body lumen such that the nozzle accelerates a fluid stream passing through the upstream component towards the downstream component to generate a low pressure region in a vicinity of the entrainment region that entrains additional fluid into the fluid stream via the one or more openings as the fluid stream passes into the downstream component.

The upstream component and the downstream component may be formed from a frame, and the flow modulator device may include a coating on at least a portion of the upstream component and the downstream component, such that the one or more openings may be defined by one or more uncoated portions of the frame. The frame may define a plurality of cells. At least a portion of a distal portion of downstream component may be uncoated, the distal portion configured to adapt with the body lumen to thereby prevent migration of the flow modulator device within the body lumen.

In some embodiment, the coating may be configured to tear upon application of force to the coating to transition the flow modulator device from an operable state where the nozzle accelerates the fluid stream passing through the upstream component towards the downstream component to generate the low pressure region in the vicinity of the entrainment region that entrains additional fluid into the fluid stream, to an inoperable state where a hemodynamic effect of the flow modulator device is effectively eliminated. For example, the coating may be configured to tear upon application of force via at least one of a cutting tool, a puncture tool, or a power-driven tool. Accordingly, the frame may be configured to transition between a contracted hourglass configuration comprising the nozzle and the diffuser in the operable state, and an expanded cylindrical configuration where fluid flow is no longer modulated through the flow modulator device in the inoperable state. The coating may comprise one or more weak lines configured to facilitate tearing of the coating upon application of force to the one or more weak lines to facilitate transitioning of the frame from the contracted hourglass configuration to the expanded cylindrical configuration. In some embodiments, the frame may be twisted between sealing zones of the flow modulator device to form the contracted hourglass configuration, and wherein the one or more weak lines are disposed along the twist between the sealing zones.

The frame may comprise a first frame forming the upstream component and a second frame forming the downstream component, such that the first frame may be coupled to the second frame via the coating. The first and second frames may be biased towards the expanded cylindrical configuration. Moreover, the coating may comprise one or more weak lines configured to facilitate tearing of the coating upon application of force to the one or more weak lines to facilitate transitioning of the first and second frames from the contracted hourglass configuration to the expanded cylindrical configuration. The flow modulator device further may include one or more fasteners configured to maintain the first and second frames in the contracted hourglass configuration. The one or more fasteners may be configured to break upon application of force to the one or more fasteners to facilitate transitioning of the first and second frames from the contracted hourglass configuration to the expanded cylindrical configuration. For example, the one or more fasteners may be configured to break via at least one of cutting, ablation, degradation, or balloon expansion. A distal end of the first frame may comprise a first set of rings configured to receive the one or more fasteners therethrough, and a proximal end of the second frame may comprise a second set of rings configured to receive the one or more fasteners therethrough. In addition, the one or more fasteners may comprise a first fastener configured to be coupled to a distal end of the first frame, and a second fastener configured to be coupled to a proximal end of the second frame to thereby maintain the first and second frames in the contracted hourglass configuration. Upon breaking of the one or more fasteners, the one or more fasteners may be configured to remain coupled to the first frame and/or the second frame to prevent emboli flow of the one or more fasteners downstream of the flow modulator device.

In some embodiments wherein the coating comprises one or more weak lines configured to facilitate tearing of the coating upon application of force to the one or more weak lines, the one or more weak lines may be disposed on the coating in a manner such that, in the inoperable state, the torn coating along the one or more weak lines forms one or more flap openings configured to effectively eliminate the hemodynamic effect of the flow modulator device. For example, the one or more weak lines may be disposed within one or more cells defined by the frame. Alternatively, the one or more weak lines may extend across one or more cells defined by the frame.

The one or more openings may include a plurality of openings circumferentially spaced around the entrainment region. The downstream component may include a third diverging portion downstream from the second diverging portion, such that the third diverging portion's average angle of divergence may be greater than the second diverging portion's average angle of divergence. The flow modulator device may be configured to transition from a collapsed delivery state to an expanded deployed state within the body lumen. In the expanded deployed state, a proximal portion of the upstream component and a distal portion of the downstream component may be configured to adapt with the body lumen. In some embodiments wherein the upstream component and the downstream component may be formed from a frame, the flow modulator device may comprise an anchor. For example, the anchor may comprise a frame portion configured to be coupled to the frame, a pair of struts comprising a downstream portion extending from the frame portion and away from each other in an upstream direction, and an upstream portion extending from the downstream portion and toward each other in the upstream direction, and a loop portion extending axially and radially outward from the frame portion in the upstream direction between the pair of struts. The loop portion may be configured engage the body lumen in the expanded deployed state to secure the flow modulator device within the body lumen. Accordingly, upon transitioning of the flow modulator device from the expanded deployed state to the collapsed delivery state, the pair of struts may be configured to move towards each other to cause the loop portion to contract radially inward, such that the loop portion does not extend radially beyond the pair of struts.

The flow modulator device further may include a retrieval portion extending from the inlet of the upstream component and converging in an upstream direction toward an upstream end of the flow modulator device. The retrieval portion may be configured to facilitate retrieval of the flow modulator device. For example, the retrieval portion may include a hook at the upstream end of the flow modulator device, the hook configured to be pulled to collapse the upstream component. The diffuser may decelerate the fluid stream having the entrained additional fluid passing through the downstream component. In addition, the downstream component's length may be greater than the upstream component's length. Moreover, the upstream component's average angle of convergence may be greater than the downstream component's average angle of divergence. The one or more openings may extend into the flow modulator device's narrowest section.

The flow modulator device further may include an adjustor disposed around a circumference of the upstream component adjacent the outlet of the upstream component, such that the adjustor may be configured to be actuated to adjust an internal diameter of the outlet *in vivo*. For example, the adjustor may comprise an annular balloon disposed around the circumference of the upstream component adjacent the outlet of the upstream component, and an access port fluidically coupled to the annular balloon via an inflation line. Accordingly, the access port may be configured to receive and expel fluid to thereby inflate and deflate the annular balloon to adjust the internal diameter of the outlet *in vivo*. Alternatively, the adjustor may comprise a band disposed around the circumference of the upstream component adjacent the outlet of the upstream component, a motor operatively coupled to the band, and an external control unit operatively coupled to the motor. Accordingly, the external control unit may be configured to actuate the motor to adjust a diameter of the band to adjust the internal diameter of the outlet *in vivo*.

In accordance with another aspect, a method, which does not form part of the present invention, for altering fluid flow through a body lumen coupled to a branch lumen is provided. The method may include: providing a flow modulator device configured to be positioned within the body lumen such that an upstream component of the flow modulator device is positioned in a first portion of the body lumen upstream of the branch lumen, a downstream component of the flow modulator device is positioned in a second portion of the body lumen downstream of the branch lumen, and an entrainment region of the flow modulator device is positioned in a vicinity of the branch lumen, the upstream component having an inlet, an outlet, and a cross-sectional flow area that converges from the inlet towards the outlet, the downstream component having an entry, an exit, and a cross-sectional flow area that diverges from the entry towards the exit, and the entrainment region comprising one or more openings extending across at least a portion of both a first diverging portion of the downstream component and a second diverging portion of the downstream component, the first diverging portion's average angle of divergence greater than the second diverging portion's average angle of divergence; receiving a fluid stream through the inlet of the upstream component; and accelerating the fluid stream passing through the upstream component towards the downstream component to generate a low pressure region in a vicinity of the entrainment region and to entrain additional fluid from the branch lumen into the fluid stream as the fluid stream passes into the downstream diffuser.

For example, the upstream component may be configured to be positioned in an inferior vena cava upstream of a branch off to a renal vein, and the downstream component may be configured to be positioned in the inferior vena cava downstream of the branch off to the renal vein, such that the entrainment region is in the vicinity of the branch off to the renal veins, thereby drawing blood from the renal veins and improving kidney functionality. Moreover, drawing blood from the renal veins to improve kidney functionality may further reduce excess fluid to treat heart failure. The flow modulator device may be configured to be delivered to the body lumen in a collapsed delivery state, and to transition from the collapsed delivery state to an expanded deployed state within the body lumen such that a proximal portion of the upstream component adapts with the first portion of the body lumen and a distal portion of the downstream component adapts with the second portion of the body lumen. The method further may include pulling a retrieval portion to transition the flow modulator device towards the collapsed delivery state, the retrieval portion extending from the inlet of the upstream component in an upstream direction toward an upstream end of the flow modulator device.

In accordance with another aspect of the present disclosure, another flow modulator device for altering fluid flow through a body lumen, the body lumen coupled to a branch lumen, is provided. The flow modulator device may comprise an upstream component having an inlet, an outlet, and a cross-sectional flow area that converges from the inlet towards the outlet to form a nozzle, a downstream component having an entry, an exit, and a cross-sectional flow area that diverges from the entry towards the exit to form a diffuser, the upstream component and the downstream component formed from a frame, a coating disposed on at least a portion of the upstream component and the downstream component, and an entrainment region between the inlet of the upstream component and the exit of the downstream component. The entrainment region may comprise one or more openings defined by one or more uncoated portions of the frame. Moreover, the coating may be configured to tear upon application of force to the coating to transition the flow modulator device from an operable state where the nozzle accelerates a fluid stream passing through the upstream component towards the downstream component to generate a low pressure region in a vicinity of the entrainment region that entrains additional fluid into the fluid stream via the one or more openings as the fluid stream passes into the downstream component, to an inoperable state where a hemodynamic effect of the flow modulator device is effectively eliminated.

The frame may define a plurality of cells, such that the frame further may comprise one or more connectors at one or more junctions between adjacent cells of the plurality of cells. The one or more connectors may be configured to facilitate transitioning of the frame from the contracted hourglass configuration to the expanded cylindrical configuration, such that a segment of the frame adjacent to the one or more connectors may maintain its overall length as the frame transitions from the contracted hourglass configuration to the expanded cylindrical configuration. In addition, the flow modulator device may include a retrieval portion extending from the inlet of the upstream component, and a hook coupled to the retrieval portion. Accordingly, the hook may be configured to be pulled to collapse the upstream component to facilitate retrieval of the flow modulator device. Moreover, the downstream component may comprise a first diverging portion and a second diverging portion downstream from the first diverging portion, the first diverging portion's average angle of divergence greater than the second diverging portion's average angle of divergence, and the one or more openings may extend across at least a portion of both the first and second diverging portions of the downstream component.

In accordance with another aspect, another method, which does not form part of the present invention, for altering fluid flow through a body lumen coupled to a branch lumen is provided. The method may include: providing a flow modulator device configured to be positioned within the body lumen such that an upstream component of the flow modulator device having an inlet, an outlet, and a cross-sectional flow area that converges from the inlet towards the outlet is positioned in a first portion of the body lumen upstream of the branch lumen, a downstream component of the flow modulator device having an entry, an exit, and a cross-sectional flow area that diverges from the entry towards the exit is positioned in a second portion of the body lumen downstream of the branch lumen, and an entrainment region of the flow modulator device comprising one or more openings is positioned in a vicinity of the branch lumen, the upstream and downstream components formed from a frame, and at least a portion of the upstream and downstream components comprising a coating, such that the one or more openings are defined by one or more uncoated portions of the frame; receiving a fluid stream through the inlet of the upstream component; and accelerating, when the flow modulator device is in an operable state, the fluid stream passing through the upstream component towards the downstream component to generate a low pressure region in a vicinity of the entrainment region and to entrain additional fluid from the branch lumen into the fluid stream as the fluid stream passes into the downstream diffuser. Moreover, the coating may be configured to tear upon application of force to the coating to transition the flow modulator device from the operable state to an inoperable state where a hemodynamic effect of the flow modulator device is effectively eliminated.

The method further may include applying force to the coating to tear the coating and transition the flow modulator device from the operable state to the inoperable state. The frame may comprise a contracted hourglass configuration comprising the nozzle and the diffuser in the operable state, and an expanded cylindrical configuration in the inoperable state. Accordingly, applying force to the coating to tear the coating and transition the flow modulator device from the operable state to the inoperable state may comprise applying force to one or more fasteners maintaining the frame in the contracted hourglass configuration to break the one or more fasteners. Moreover, applying force to the one or more fasteners to break the one or fasteners may comprise applying force via at least one of cutting, ablation, degradation, or balloon expansion. In some embodiments, the frame may be twisted between one or more sealing zones to form the contracted hourglass configuration. Accordingly, applying force to the coating to tear the coating and transition the flow modulator device from the operable state to the inoperable state may comprise applying force to the coating to tear the coating along one or more weak lines disposed on the one or more sealing zones.

In addition, applying force to the coating to tear the coating and transition the flow modulator device from the operable state to the inoperable state may comprise applying force to the coating to tear the coating along one or more weak lines disposed on the coating to form one or more flap openings configured to eliminate the hemodynamic effect of the flow modulator device. Further, applying force to the coating to tear the coating may comprise applying force via at least one of a cutting tool, a puncture tool, or a power-driven tool. The method further may include adjusting an internal diameter of the outlet of the upstream component *in vivo.* For example, adjusting an internal diameter of the outlet of the upstream component *in vivo* may comprise inflating or deflating an annular balloon disposed around the circumference of the upstream component adjacent the outlet of the upstream component via an access port fluidically coupled to the annular balloon via an inflation line. Alternatively, adjusting an internal diameter of the outlet of the upstream component *in vivo* may comprise actuating a motor operatively coupled to a band disposed around the circumference of the upstream component adjacent the outlet of the upstream component to adjust a diameter of the band.

BRIEF DESCRIPTION OF THE DRAWINGS

FIG. 1 is a side view of a fluid flow modulator.

FIGS. 2A and 2B are side views of an exemplary fluid flow modulator constructed in accordance with the principles of the present invention.

FIG. 2C illustrates the angles of divergence of first and second diverging portions of the fluid flow modulator of FIG. 2B in accordance with the principles of the present disclosure.

FIG. 3 illustrates the fluid flow modulator of FIG. 2A disposed within a body lumen coupled to a branch lumen in accordance with the principles of the present disclosure.

FIG. 4A illustrates entrained fluid flow through the fluid flow modulator of FIG. 2A compared with entrained fluid flow through the fluid flow modulator of FIG. 1.

FIG. 4B is a cross-sectional view of the entrained fluid flow of FIG. 4A.

FIG. 5 illustrates the frame structure of the fluid flow modulator of FIG. 2A.

FIG. 6 is a graph illustrating kidney function versus renal blood flow in accordance with the principles of the present disclosure.

FIG. 7 is a graph illustrating population survival of patients having various glomerular filtration rates.

FIG. 8A is a graph illustrating the effects of a flow modulator on urine output before, during, and after treatment.

FIG. 8B is a graph illustrating the effects of a flow modulator on total Natriuresis, before, during, and after treatment.

FIGS. 9A and 9B are side views of an alternative exemplary fluid flow modulator constructed in accordance with the principles of the present disclosure.

FIGS. 10A to 10C are side views of another alternative exemplary fluid flow modulator constructed in accordance with the principles of the present disclosure.

FIGS. 11A to 11C are side views of another alternative exemplary fluid flow modulator constructed in accordance with the principles of the present disclosure.

FIG. 12A is a side view of another alternative exemplary fluid flow modulator constructed in accordance with the principles of the present disclosure.

FIG. 12B illustrates the frame structure of the fluid flow modulator of FIG. 12A before expansion.

FIG. 12C illustrates the frame structure of the fluid flow modulator of FIG. 12A after expansion.

FIG. 13A is a perspective view of a non-elongated hook assembly for a fluid flow modulator in a non-assembled configuration, constructed in accordance with the principles of the present disclosure.

FIG. 13B is a perspective view of the non-elongated hook assembly of FIG. 13A in an assembled configuration.

FIG. 13C is a side view of the non-elongated hook assembly of FIGS. 13A and 13B in a radially compressed configuration.

FIG. 13D is an upstream view of the non-elongated hook assembly in the non-assembled configuration of FIG. 13A.

FIGS. 13E and 13F are upstream views of the non-elongated hook assembly of FIGS. 13B and 13D as the diameter of the sealing zone of the flow modulator changes in accordance with the principles of the present disclosure.

FIG. 14 is a side view of an elongated hook assembly constructed in accordance to with the principles of the present disclosure.

FIG. 15A is a perspective view of a crimpable anchoring feature for a fluid flow modulator constructed in accordance with the principles of the present disclosure.

FIG. 15B is a side view of the crimpable anchoring feature of FIG. 15A in use with a fluid flow modulator constructed in accordance with the principles of the present disclosure.

FIG. 15C is a side view of the crimpable anchoring feature of FIGS. 15A and 15B in use with a fluid flow modulator and a retrieval tool.

FIG. 16A is a cross-sectional view of a delivery system sheath with a retracted fluid flow modulator constructed in accordance with the principles of the present disclosure.

FIG. 16B is a cross-sectional view of the delivery system sheath of FIG. 16A with a partially deployed fluid flow modulator.

FIG. 17A is a side view of an adjustable nozzle balloon for a fluid flow modulator constructed in accordance with the principles of the present disclosure.

FIG. 17B is a side view of the adjustable nozzle balloon of FIG. 17A in use with a fluid flow modulator disposed within a body lumen, wherein the adjustable nozzle balloon is operatively coupled to a control unit.

FIG. 18A is a perspective view of an adjustable nozzle band for a fluid flow modulator constructed in accordance with the principles of the present disclosure.

FIG. 18B is a perspective view of the adjustable nozzle band of FIG. 18A in use with a fluid flow modulator.

FIG. 18C is side view of the fluid flow modulator having the adjustable nozzle band of FIG. 18B disposed within a body lumen, wherein the adjustable nozzle band is operatively coupled to a control unit.

FIG. 19A illustrates a frame structure utilizing exemplary connectors in a compact configuration, constructed in accordance with the principles of the present disclosure.

FIG. 19B illustrates a frame structure utilizing the connectors of FIG. 19A in an expanded configuration.

## DETAILED DESCRIPTION OF EMBODIMENTS

Devices and methods for altering flow in body lumens are provided for creating pressure differences and/or to induce fluid entrainment from branch lumens for enhancing or modifying fluid flow to treat different disorders or diseases.

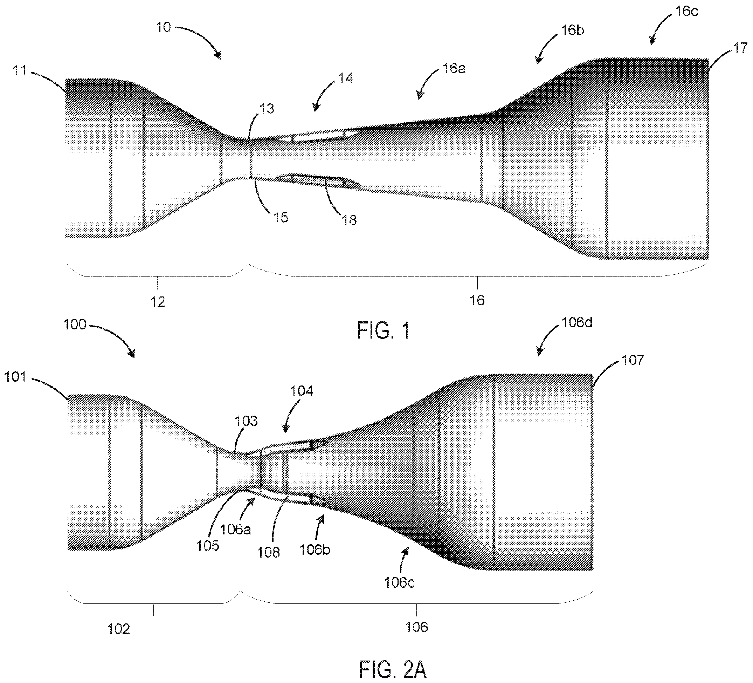

Referring to FIG. 1, flow modulator 10 constructed and operative in accordance with the principles of the present disclosure is provided. Flow modulator 10 may be constructed similar to the flow modulators described in, for example, PCT International Patent Application Publications WO 2016/128983, WO 2018/029688, WO 2018/220589, WO 2019/097424, and WO 2020/109979, PCT/IB2022/060573, U.S. Patent Nos. 10,195,406 and 11,324,619, and U.S. Patent Application Publication No. 2022/0039938. For example, as shown in FIG. 1, flow modulator 10 may have upstream component 12, downstream component 16, and an entrainment region 14 disposed between upstream component 12 and downstream component 16. Flow modulator 10 is sized and shaped to be implanted in a body lumen, and may be compressed for delivery (e.g., percutaneous delivery within a delivery sheath) and expanded upon deployment (e.g., self-expanding upon release from the end of the delivery sheath or balloon expandable).

Entrainment region 14 may be integrally formed in downstream component 16 as shown in FIG. 1, or alternatively in upstream component 12, or both. Entrainment region 14 may include one or more openings 18 designed to entrain fluid into a stream of fluid flowing from upstream component 12 to downstream component 16. Upstream component 12 and downstream component 16 create a lower pressure region in the vicinity of entrainment region 14, which preferably entrains fluid into the stream of fluid flowing across entrainment region 14. Fluid entrainment is induced by shear-induced turbulent flux. In accordance with the principles of the disclosure, such entrainment is expected to transport blood or other body fluids to or from a region so as to improve organ function (e.g., from the renal vein(s) to the inferior vena cava to promote better functionality of the kidney(s) and/or from the hepatic vein(s) to the inferior vena cava to improve liver function, thereby treating disorders and/or diseases such as heart failure).

Upstream component 12 has inlet 11 and outlet 13, and has a cross-sectional flow area that converges in a downstream direction, e.g., from upstream component 12 towards downstream component 16, along part or all of the length of upstream component 12, thereby forming a nozzle. In this manner, upstream component 12 accelerates flow of fluid through upstream component 12. Downstream component 16 has entry 15 and exit 17, and has a cross-sectional flow area that diverges in a downstream direction along part or all of the length of downstream component 16, thereby forming a diffuser.

As shown in FIG. 1, downstream component 16 may include first diverging portion 16a, second diverging portion 16b downstream from first diverging portion 16a, and portion 16c downstream from second diverging portion 16b. The average angle of divergence of second diverging portion 16b preferably is greater than the average angle of divergence of first diverging portion 16a. As shown in FIG. 1, openings 18 may extend longitudinally along a proximal portion of first diverging portion 16a, e.g., adjacent to the narrowest portion of downstream component 16, such that the angle of divergence of entrainment region 14 is constant along openings 18. Portion 16c may include a proximal sealing zone, which contacts the wall of the body lumen in its expanded deployed state, and a distal portion downstream of the sealing zone, which may adapt to the vessel without damaging the vessel to prevent migration of flow modulator 10. Downstream component 16 thus decelerates flow of fluid through downstream component 16. The length of entrainment region 14 may be selected to generate a low pressure region in the vicinity of entrainment region 14, while minimizing pressure loss and reducing resistance to fluid flow from the branch lumen(s), e.g., renal flow.

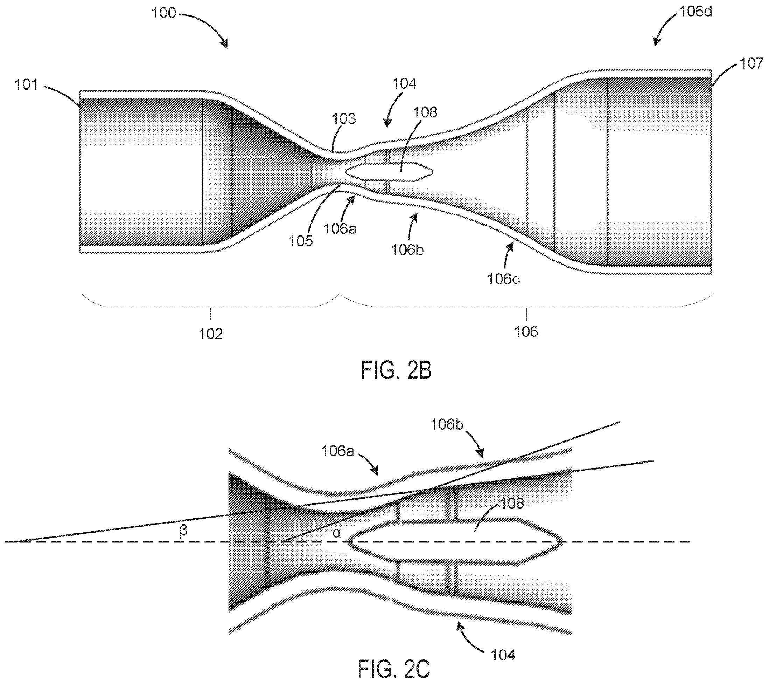

Referring now to FIGS. 2A to 2C, an optimized flow modulator constructed and operative in accordance with the principles of the present invention is provided. Flow modulator 100 may be constructed similar to flow modulator 10. For example, as shown in FIGS. 2A and 2B, flow modulator 100 may have upstream component 102, downstream component 106, and an entrainment region 104 disposed between upstream component 102 and downstream component 106, e.g., on at least a proximal portion of downstream component 106. Like flow modulator 10, flow modulator 100 is sized and shaped to be implanted in a body lumen, and may be compressed for delivery (e.g., percutaneous delivery within a delivery sheath) and expanded upon deployment (e.g., self-expanding upon release from the end of the delivery sheath or balloon expandable).

Entrainment region 104 may be integrally formed in downstream component 106 as shown in FIGS. 2A to 2C, or alternatively in upstream component 102, or both. Entrainment region 104 may include one or more openings 108 designed to entrain fluid into a stream of fluid flowing from upstream component 102 to downstream component 106. Upstream component 102 and downstream component 106 create a lower pressure region in the vicinity of entrainment region 104, which preferably entrains fluid into the stream of fluid flowing across entrainment region 104. Fluid entrainment is induced by shear-induced turbulent flux. In accordance with the principles of the invention, such entrainment is expected to transport blood or other body fluids to or from a region so as to improve organ function (e.g., from the renal vein(s) to the inferior vena cava to promote better functionality of the kidney(s) and/or from the hepatic vein(s) to the inferior vena cava to improve liver function, thereby treating disorders and/or diseases such as heart failure).

Upstream component 102 has inlet 101 and outlet 103, and has a cross-sectional flow area that converges in a downstream direction, e.g., from upstream component 102 towards downstream component 106, along part or all of the length of upstream component 102, thereby forming a nozzle. In this manner, upstream component 102 accelerates flow of fluid through upstream component 102. Downstream component 106 has entry 105 and exit 107, and has a cross-sectional flow area that diverges in a downstream direction along part or all of the length of downstream component 106, thereby forming a diffuser.

As shown in FIGS. 2A and 2B, downstream component 106 may include first diverging portion 106a, second diverging portion 160b downstream from first diverging portion 106a, third diverging portion 106c downstream from second diverging portion 106b, and portion 106d downstream from third diverging portion 106c. The average angle of divergence of first diverging portion 106a preferably is greater than the average angle of divergence of second diverging portion 106b, and the average angle of divergence of third diverging portion 106c preferably is greater than the average angle of divergence of second diverging portion 106b. For example, as shown in FIG. 2C, first diverging portion 106a has average angle of divergence $\alpha$, and second diverging portion 106b has average angle of divergence $\beta$, which is smaller than average angle of divergence $\alpha$. Average angle of divergence $\alpha$ of first diverging portion 106a may be in a range from, e.g., 10-75 degrees, and average angle of divergence $\beta$ of second diverging portion 106b of downstream component 106 and may be in a range from, e.g., 2-30 degrees, for example, 3-10 degrees. In FIG. 2C, $\alpha$ is approximately 25 degrees and $\beta$ is approximately 4 degrees.

As shown in FIG. 2C, openings 108 may extend longitudinally along at least a portion of first diverging portion 106a, e.g., adjacent to the narrowest portion of downstream component 106, and at least a portion of second diverging portion 106b, such that the angle of divergence of entrainment region 104 transitions from an average angle of divergence $\alpha$ to an average angle of divergence $\beta$ in the downstream direction. For example, the upstream end of openings 108 may be disposed immediately downstream of outlet 103 of upstream component 102, e.g., at entry 105 of downstream component 106. Alternatively, the upstream end of openings 108 may be disposed, e.g., 0-5 mm downstream of outlet 103 of upstream component 102. As described in further detail below, the average angle of divergence $\alpha$ of openings 108 extending along first diverging portion 106a permits an additional amount of entrained fluids through openings 108 as fluid flow is accelerated through flow modulator 100 from upstream component 102 through downstream component 106.

Portion 106d may include a proximal sealing zone, which contacts the wall of the body lumen in its expanded deployed state, and a distal portion downstream of the sealing zone, which may adapt to the vessel without damaging the vessel to prevent migration of flow modulator 100 during, e.g., coughing or other events that may cause a dramatic change in vessel diameter. Downstream component 106 thus decelerates flow of fluid through downstream component 106. The length of entrainment region 104, e.g., the length of openings 108 extending along first diverging portion 106a and the length of openings 108 extending along second diverging portion 106b, may be selected to generate a low pressure region in the vicinity of entrainment region 104, while minimizing pressure loss and reducing resistance to fluid flow from the branch lumen(s), e.g., renal flow. Other converging and diverging structures suitable for use in accordance with the principles of the present disclosure are described herein.

The central axis of the diverging portion may be disposed in-line with, or offset from, the central axis of the converging portion. As shown in FIGS. 2A and 2B, upstream component 102 and downstream component 106 share common, collinear flow axis. Alternatively, upstream component 102 may be angled with respect to downstream component 106. Upstream component 102 and downstream component 106 also may lie along a continuously curved path.

Upstream component 102 and downstream component 106 may be constructed as grafts, stents (coated or uncoated), stent grafts (coated or uncoated), and the like, and are formed of biocompatible materials, such as stainless steel or Nitinol. The outer contours of any of upstream component 102 and downstream component 106 may be sealed against the inner wall of the body lumen (such as by being expanded thereagainst), or alternatively may not be sealed, depending on the particular application. This may be referred to as the fixation area(s).

Flow modulator 100 may be inserted into the body lumen in an antegrade or retrograde manner and similarly may be removed antegrade or retrograde. Flow modulator 100 may be used as an acute device to be removed after few hours/days or a chronic permanent device or a device that can be retrieved after long-term implantation. Additionally, flow modulator 100 may be decoupled from the delivery device and left in the patient for, e.g., 1-5 days or preferably 3 days, before retrieval and removal from the patient's body. When used as an acute device, flow modulator 100 may remain coupled to a delivery/retrieval device, e.g., sheath and/or wire/shaft, throughout the short-term implantation for ease of device delivery and retrieval. Flow modulator 100 may be compressible while disposed within a body lumen to allow periodic wash-out of stagnant flow zones created adjacent to flow modulator 100. For example, flow modulator 100 may be partially or fully reduced in diameter within the body lumen to allow blood flow through a stagnant flow zone.

Preferably, upon expansion, flow modulator 100 is sized to contact the inner wall of the body lumen to anchor flow modulator 100 within the lumen. Specifically, upstream component 102 may have a fixation area sized for anchoring upstream component 102 within the body lumen in its expanded, deployed state. For example, the fixation area of upstream component 102 may be sized to contact the inner wall of the body lumen and preferably has a diameter the size of, or slightly larger than, the diameter of the body lumen. The fixation area of upstream component 102 may have a constant diameter for a length suitable for anchoring upstream component 102 in the body lumen. Similarly, downstream component 106 may have a fixation area sized for anchoring downstream component 106 within another portion of the body lumen. For example, the fixation area of downstream component 106 may include at least a portion of third diverging portion 106c and/or uncovered portion 106d of downstream component 106. The fixation area of downstream component 106 may be sized to contact the inner wall of the other portion of the body lumen and preferably has a diameter the size of, or slightly larger than, the diameter of that portion of the body lumen. The fixation area of downstream component 106 may have a constant diameter for a length suitable for anchoring downstream component 106 in the body lumen. Preferably the fixation areas of upstream component 102 and downstream component 106 are configured to seal fluid modulator 100 within the body lumen so that fluid only flows into the fluid channels created by fluid modulator 100 and does not flow between the fixation areas of upstream component 102 and downstream component 106 and the vessel wall.

Although the invention is not bound by any theory, a simplified engineering explanation is now provided to help understand how upstream component 102 and downstream component 106 operate to create reduced pressure at entrainment region 104.

The Bernoulli equation governs the relationship between fluid velocity and pressure (neglecting the height difference):

$$P_1 + \frac{1}{2} \cdot \rho \cdot V_1^2 = P_2 + \frac{1}{2} \cdot \rho \cdot V_2^2 + E_{loss}$$

P = pressure
$\rho$ = density
V= velocity
1= conditions at the inlet (upstream component 102)
2= conditions at entrainment region 104

Mass conservation (same flow rate):

$$V_1 \cdot A_1 = V_2 \cdot A_2$$

A= Flow cross section
$E_{loss}$ = Energy loss

For example, if flow modulator 100 is installed near the kidneys with upstream component 102 in the inferior vena cava, then $V_1$ and $A_1$ are the velocity and flow area, respectively, at the inferior vena cava.

The flow velocity at the entrainment region ($V_2$) is designed to achieve the desired pressure reduction. For example, for 0.5 meter per second velocity and 3 times area ratio, a suction of about 6-8 mm Hg can be achieved. In the case of deployment near the kidney, this pressure differential is expected to improve renal function by improving renal perfusion pressure. The pressure will change due to improvement in the renal flow.

Applicant has discovered that using a maximum length of openings 108, and having at least a portion of openings 108 extend along first diverging portion 106a having an average angle of divergence that is greater than the average angle of divergence of the portion of openings 108 extending along second diverging portion 106b will improve flow rates in the branched vessel(s) with relatively low pressure loss. A length too large will create a significant pressure loss that actually sends flow in the wrong direction in the renal vein(s). In addition, other structural characteristics of the downstream component improve renal flow with low pressure loss such as a greater inner diameter at the entry of the downstream component than the inner diameter at the outlet of the upstream component, a greater length of the diverging area of the downstream component than the length of the converging area of the upstream component, and/or a lesser average angle of divergence of the downstream component than the average angle of convergence of the upstream component.

In another example, flow modulator 100 may be installed near a bifurcation to divert emboli from the bifurcation. In yet another example, flow modulator 100 may be deployed in the aortic arch to reduce peak systolic pressure.

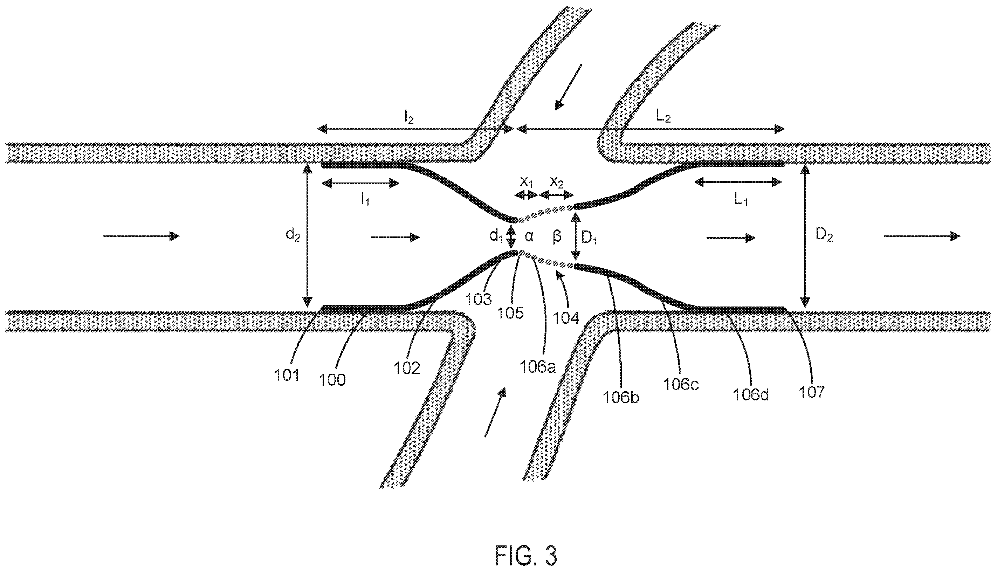

Referring now to FIG. 3, flow modulator 100 is shown disposed within a body lumen coupled to a branch lumen with symbols depicting dimensions of flow modulator 100 in accordance with a preferred embodiment. The dimensions provided with respect to FIG. 3 are for an embodiment suitable for implantation in the inferior vena cava. In particular, inlet 101 of upstream component 102 is configured to be disposed upstream from a branch off to a renal vein(s), downstream component 106 is configured to be disposed in the inferior vena cava, such that exit 107 is downstream from the branch off to the renal vein(s), and entrainment region 104 is in the vicinity of the branch to the renal vein(s). $d_1$ is the diameter of outlet 103 of upstream component 102, which is leads into entry 105 of downstream component 106. $d_1$ is selected to create a jet velocity for a given device resistance. In the example of chronic cases, $d_1$ may be in a range from 3.5-8 mm. In acute cases, $d_1$ preferably is in a range from 3-7 mm. $d_2$ is the diameter of inlet 101 in the deployed, expanded state and may be in a range from 12-45 mm. $l_1$ is the length of the fixation area of upstream component 102, and may be in a range from 5-30 mm. $l_2$ is the overall length of upstream component 102 and may be in a range from 15-60 mm.

$x_1$ is the length of openings 108 extending along first diverging portion 106a, and $x_2$ is the length of openings 108 extending along second diverging portion 106b, such that the sum of $x_1$ and $x_2$ is the axial length of entrainment region 104. For the length of entrainment region 104, a shorter length may provide better performance for downstream component 106, but will result in lower renal flow because there is a greater resistance to flow from the renal vein(s) to downstream component 106. Thus, the length of entrainment region 104 preferably is selected to provide improved renal flow rate with minimal pressure loss. Preferably, $x_2$ is larger than $x_1$. For example, the ratio of $x_2$:$x_1$ may be from 1:1 to 5:1.

Still referring to FIG. 3, $L_1$ is the length of the fixation area of downstream component 106, and may be in a range from 5-30 mm. $L_2$ is the overall length of downstream component 106. $L_2$ is preferably greater than $l_2$ because a diverging shape creates a much higher pressure loss than a converging shape. The ratio of $L_2$:$l_2$ may be from 1:1 to 3:1. $D_1$ is the diameter of downstream component 106 at the downstream end of entrainment region 104, and is preferably larger than $d_1$. Thus, the cross-sectional flow area at outlet 103 of upstream component 102 and entry 105 of downstream component 106 is less than the cross-sectional flow area at the downstream end of entrainment region 104 of downstream component 106. $D_1$ is selected to receive all the fluid jetted from outlet 103, as well as the additional fluid entrained through openings 108. The ratio of $D_1$:$d_1$ may be from 1:1 to 3.5:1. In addition, $D_1$ should be greater for larger lengths of entrainment region 104 to ensure receipt of the fluid jetted from upstream component 102. $D_2$ is the diameter of exit 107 in the deployed, expanded state and may be in a range from 12-45 mm. The ratio of $D_2$:$d_2$ may be from 1:1 to 1.75:1. For example, when $D_2$ is 30 mm, $d_2$ may be 20 mm.

Preferably, the angle of divergence in downstream component 106 is less than the angle of convergence in upstream component 102, and is expected to prevent pressure loss. In FIG. 3, $\alpha$ is the average angle of divergence of first diverging portion 106a of downstream component 106 and may be in a range from, e.g., 10-75 degrees, and $\beta$ is the average angle of divergence of second diverging portion 106b of downstream component 106 and may be in a range from, e.g., 2-30 degrees, for example, 3-10 degrees. As shown in FIG. 3, average angle of divergence $\alpha$ is greater than average angle of divergence $\beta$. A quick change in the cross-sectional flow area across first diverging portion 106a is preferred to maximize the additional amount of fluid entrained through openings 108 along first diverging portion 106a. As described above, the length of openings 108 extending along first diverging portion 106a may be less than or equal to the length of openings 108 extending along second diverging portion 106b.

In addition, downstream component 106 should have slow change in area adjacent to and downstream from first diverging portion 106a, along second diverging portion 106b - any additional pressure loss will reduce the inferior vena cava flow rate and thus will reduce the effectiveness of the device. The angle of divergence in second diverging portion 106b of downstream component 106 may be constant or may change along the length of downstream component 106. When the angle of divergence changes along the length, the angle of divergence is preferably smallest (e.g., in a range from 5-30 degrees) adjacent to the downstream end of first diverging portion 106a. A slow change in the cross-sectional flow area along second diverging portion 106b is preferred to reduce flow separation and thus, reduce pressure loss, as fluid velocity decreases as the cross-sectional flow area increases.

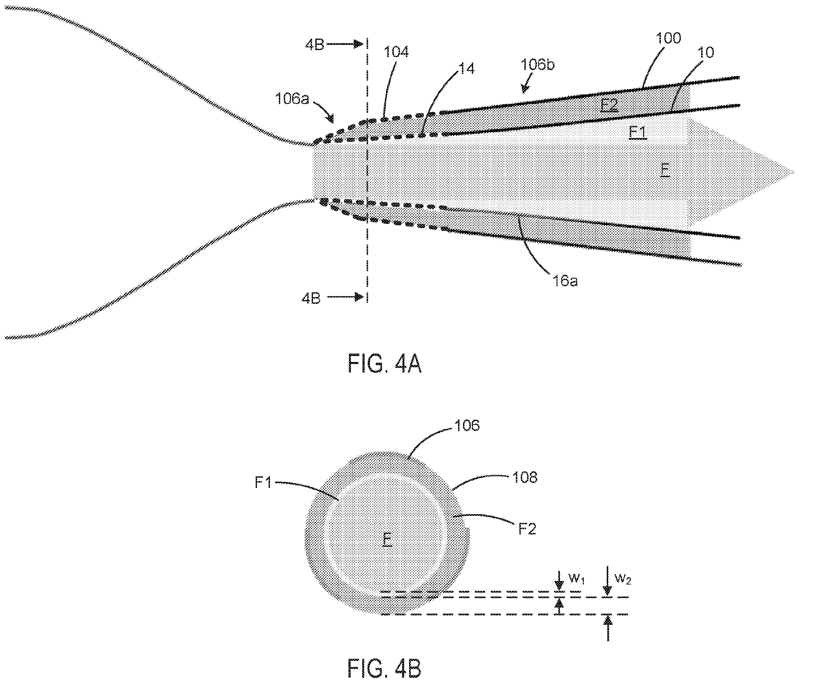

Referring now to FIGS. 4A and 4B, fluid flow through flow modulator 100 compared with fluid flow through fluid modulator 10 is provided. As shown in FIG. 4A, IVC jet flow F flows through the flow modulator, e.g., flow modulator 10 or flow modulator 100, from the upstream nozzle through the downstream diffuser. As IVC jet flow F is accelerated through the upstream nozzle and decelerates through the downstream diffuser, a low pressure region is generated in a vicinity of the entrainment region, e.g., entrainment region 14 or entrainment region 104, which causes additional flow, e.g., renal blood flow, to be entrained through the one or more openings of the entrainment region and enter the downstream diffuser. As the downstream diffuser diverges along its length from the entry of the downstream diffuser, e.g., across the entrainment region, more renal blood flow is permitted to enter into the downstream diffuser without competing for space with IVC jet flow F.

For example, as shown in FIG. 4A, entrained renal blood flow F1 enters the downstream diffuser of flow modulator 10 via the one or more openings at entrainment region 14 without competing for space with IVC jet flow F. As entrainment region 14 of flow modulator 10 has a constant angle of divergence along its length, entrained renal blood flow F1 may enter the downstream diffuser and surround IVC jet flow F, creating a "ring" flow having a width, e.g., width $w_1$ in FIG. 4B, which increases gradually through the downstream diffuser corresponding with the average angle of divergence of the downstream diffuser of flow modulator 10.

In contrast, as entrainment region 104 of flow modulator 100 has a first average angle of divergence along first diverging portion 106a and a second average angle of divergence along second diverging portion 106b, which is less than the first average angle of divergence, entrained renal blood flows F1 and F2 enter the downstream diffuser of flow modulator 100 via the one or more openings at entrainment region 104 without competing for space with IVC jet flow F. As the upstream portion of entrainment region 104 has a first average angle of divergence that is larger than the second average angle of divergence of the downstream portion of entrainment region 104, the diameter of the downstream diffuser of flow modulator 100 at the downstream end of first diverging portion 106a is larger than the diameter at the same longitudinal point along the downstream diffuser of flow modulator 10, as shown in FIG. 4A. Specifically, the diameter of the downstream diffuser of flow modulator 100 is defined by the coated portions of downstream component 106 having uncoated portions defining openings 108, as shown in FIG. 4B. Moreover, the larger angle of divergence of first diverging portion 106a provides an increased surface area for receiving a larger volume of side flow, e.g., renal blood flow entering from the renal pocket, which creates a larger space for renal blood flow to enter the downstream diffuser without competing for space with IVC jet flow F. Accordingly, during higher renal blood flow rates, there is less resistance for the renal blood flow, thereby allowing better hydrodynamic efficacy at high renal blood flow conditions. Additionally, the quick change in the cross-sectional flow area across openings 108 at first diverging portion 106a adjacent to the highest speed of IVC jet flow F provides more interaction between the fluid area and the high speed IVC jet flow,
is preferred to maximize the additional amount of fluid entrained through openings 108 along first diverging portion 106a.

Accordingly, entrained renal blood flow F1 and F2 may enter the downstream diffuser via one or more openings 108 along both first diverging portion 106a and second diverging portion 106b and surround IVC jet flow F, creating a "ring" flow having a width, e.g., the sum of width $w_1$ and $w_2$ in FIG. 4B, which increases gradually through the downstream diffuser corresponding with the first average angle of divergence of first diverging portion 106a of the downstream diffuser of flow modulator 100, and then gradually through the downstream diffuser corresponding with the second average angle of divergence of second diverging portion 106b of the downstream diffuser of flow modulator 100. As more entrained renal blood flow enters the downstream diffuser of flow modulator 100 over a given length of the downstream diffuser of flow modulator 100 compared with flow modulator 10, the length of the downstream diffuser of flow modulator 100 may be shorter than the length of the downstream diffuser of flow modulator 10 in order to entrain the same amount of blood therein.

Flow modulator 100 may be formed from one or more frames and may be coated with one or more biocompatible materials. For example, the frame(s) may be formed of a metal (e.g., shape memory metal) or alloy or a combination thereof (e.g., a stent made of stainless steel or Nitinol or cobalt chromium). For some applications, the frame(s) may include a braided stent. In the case of more than one frame, the frames may be joined together by a suitable technique, such as welding. For example, upstream component 102 and downstream component 106 may be formed from a common frame or two frames that may be joined prior to implantation.

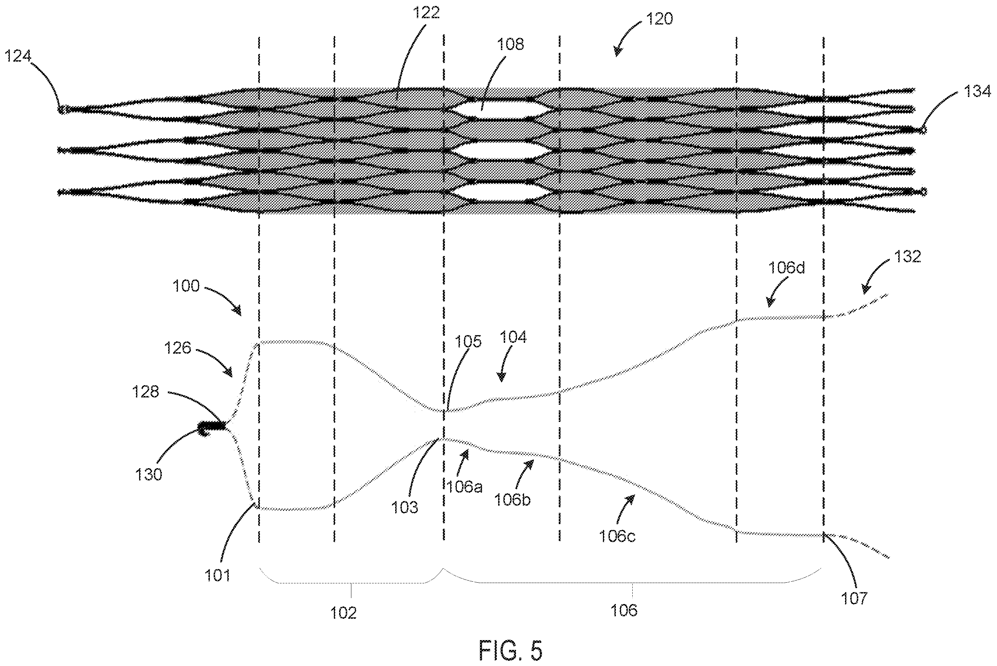

Referring now to FIG. 5, flow modulator 100 may be formed from a single frame structure 120. FIG.

5 illustrates frame 120 as cut and flattened to show the frame cutting pattern. Illustratively, upstream component 102 and downstream component 106 are defined by frame 120. Frame 120 is preferably formed from a metal tube that is laser cut to define a plurality of cells and then processed (e.g., heated) to form the shape of flow modulator 100. Flow modulator 100 may be at least partially coated with biocompatible material 122. For example, frame 120 of flow modulator 100 may initially be entirely coated with biocompatible material 122, and then selected portions of the coating may be removed, e.g., via cutting, melting, laser, chemical, etc. As shown in FIG. 5, flow modulator 100 may be only partially covered with biocompatible material 122 such that a plurality of cells upstream of inlet 101, a plurality of cells forming uncovered portion 106d, and a plurality of cells at entrainment region 104 remain uncoated.

Specifically, upstream component 102 may be coated with biocompatible material 122 to define the fluid flow channel through upstream component 102, such that fluid flowing through a body lumen enters inlet 101, accelerates through the converging portion of upstream component 102, and exits out outlet 103 and into entry 105 of downstream component 106 through entrainment region 104 of fluid modulator 100 having openings 108, e.g., uncoated portions of frame 120 at entrainment region 104. A low pressure region is formed at entrainment region 104 by the shapes of upstream component 102 and downstream component 106. Additional fluid from the branch lumen(s) at entrainment region 104 is entrained into the fluid stream passing from upstream component 102 to downstream component 106, via plurality of openings 108 formed by the uncoated portions at entrainment region 104. As shown in FIG. 5, frame 120 may be uncoated at portions evenly spaced apart extending circumferentially about entrainment region 104 of flow modulator 100, to thereby form openings 108 of flow modulator 100. As described above, the uncoated portions of frame 120 forming openings 108 may extend at least partially along both first diverging portion 106a and second diverging portion 106b. As will be understood by a person having ordinary skill in the art, although only three openings are shown in FIG. 5, entrainment region 104 may have less or more than three openings, e.g., two openings, four openings, five openings, six openings, etc., preferably evenly spaced circumferentially around entrainment region 104 of flow modulator 100.

Downstream component 106 also may be coated with biocompatible material 122 to define the fluid flow channel through downstream component 106, e.g., first diverging portion 106a, second diverging portion 106b, and third diverging portion 106c, such that the fluid stream from outlet 103 together with the additional fluid passing through plurality of openings 108 at entrainment region 104 enter downstream component 106, decelerate through the diverging portion of downstream component 106, and exit out exit 107 back into the body lumen, e.g., across uncovered portion 106d, which may remain uncoated as described in further detail below.

Biocompatible material 122 may be a fabric and/or polymer such as expanded polytetrafluoroethylene (ePTFE), woven, knitted, and/or braided polyester, polyurethane, DACRON (polyethylene terephthalate), silicone, polycarbonate urethane, or pericardial tissue from an equine, bovine, or porcine source. The biocompatible coating may impede or block fluid flow where applied to the frame. The order of the joining and coating processes may be joining before coating or coating before joining. Biocompatible material 122 may be coupled to the frame(s) via stitching, spray coating, encapsulation, electrospinning, dip molding, and/or a different technique. In some embodiments, biocompatible material 122 may be expandable, at least along some portions of flow modulator 100, to thereby adjust the cross-sectional area of the flow path through flow modulator 100 responsive to the pressure gradient across flow modulator 100, as described in further detail below.

Alternatively, flow modulator 100 may be coated with a hydrophilic, hemocompatible coating (active such as heparin coating or passive) or a drug coating. In addition, flow modulator 100 may be selectively coated in different areas. For example, flow modulator 100 may include a drug coating on the sealing zones (the portions of flow modulator 100 that contact tissue) to prevent tissue adhesion to the IVC wall, and a heparin coating on the portions of flow modulator 100 where there is constant contact with blood to thereby prevent thrombus formation.

In a preferred embodiment, biocompatible material 122 is fluid impermeable. However, for some applications, the surfaces need not be impermeable, but may have a permeability that is sufficiently low as to substantially prevent blood from flowing through the longitudinal portion of the body lumen via any flow path other than through the flow channel defined by the inner surfaces of flow modulator 100. For some applications, each of the surfaces has permeability per unit length of less than 0.25 micrometers (e.g., between 0 and 0.25 micrometers), where the permeability per unit length is defined based upon the following equation, which is based upon Darcy's Law: $k/\Delta x = V\mu/\Delta p$, where k is permeability, $\Delta x$ is length (in meters), V is average velocity (in meters per second), $\mu$ is fluid viscosity (measured in Pascal-seconds), and $\Delta P$ is the pressure differential measured in Pascals).

As shown in FIG. 5, frame 120 may define a plurality of cells, and may be heat set on a mandrel to form retrieval portion 126, upstream component 102, first diverging portion 106a, second diverging portion 106b, and third diverging portion 106c, and portion 106d having uncovered portion 132. As shown in FIG. 5, retrieval portion 126 is illustratively formed from a first plurality of cells of frame 120 upstream of upstream component 102, e.g., with no junctions from eyelets 124 to the sealing zone of upstream component 102 to thereby prevent flow disruption. For example, frame 120 may include straight struts extending from eyelets 124 toward inlet 101 without any junctions therebetween. Upstream component 102 is illustratively formed from a second plurality of cells, and downstream component 106 may be formed from additional configurations of cells, e.g., as described in U.S. Patent No. 11,324,619.

Retrieval portion 126 at the proximal end of upstream component 102 may be configured to facilitate retrieval of flow modulator 100. Retrieval portion 126 may include constricted section 128 at an upstream end of flow modulator 100 where eyelets 124 meet in the expanded, deployed state. Constricted section 128 allows flow modulator 100 to remain coupled to a delivery system. In the expanded, deployed state, the cross-sectional area of retrieval portion 126 converges from inlet 101 to constricted section 128, where eyelets 124 of retrieval portion 126 are coupled together near the center of the flow path. Retrieval portion 126 preferably is uncoated such that a fluid stream flows across the retrieval portion 126 and through inlet 101 into upstream component 102. Moreover, uncoated retrieval portion 126 may optionally serve as a filter, e.g., against thrombus and/or emboli in blood. As described above, the overall length of downstream component 106 is preferably greater than the overall length of upstream component 102 (not including retrieval portion 126). Thus, the length from inlet 101 to outlet 103 of upstream component 102 may be less than the length from entry 105 to exit 107 of downstream component 106.

As shown in FIG. 5, a retrieval device, e.g., hook 130, may be coupled to constricted portion 128 to pull retrieval portion 126 towards a delivery sheath to compress flow modulator 100 into the delivery sheath for retrieval as described in further detail below. Hook 130 may be coupled to constricted portion 128 as a separate component that is, e.g., molded, glued, compressed, welded, etc. to frame 120. In this manner a retriever, e.g., a hook or goose-neck snare device, may be coupled to hook 130 to pull retrieval portion 126 towards a delivery sheath to compress flow modulator 100 into the sheath for retrieval. Additionally, hook 130 may be pulled in a direction(s) away from entrainment region 104 to partially or fully reduce the diameter of flow modulator 100 within a body lumen. Such reduction would allow for wash-out of any stagnant flow zones created adjacent to flow modulator 100. Flow modulator 100 could then be fully removed, repositioned within the body lumen and expanded, or expanded in the prior deployment location within the body lumen. Additionally or alternatively, flow modulator 100 may include a retrieval portion at the distal end of downstream component 106 to facilitate retrieval of flow modulator 100, e.g., via the jugular vein.

Advantageously, after implantation, the flexible regions can change in diameter responsive to changes in vessel diameter while the more rigid portion of the stent structure remains constant. For example, the maximum outer diameter of upstream component 102 and downstream component 106 may change in diameter responsive to changes in vessel diameter while the shape of the outlet of the nozzle of upstream component 102 and/or the intermediate section (e.g., first diverging portion 106a and second diverging portion 106b) of flow modulator 100 does not change. In this manner, the angles of divergence of first diverging portion 106a and second diverging portion 106b may remain unchanged even though the size of the vessel changes. The change in diameter in the vessel may be measured with, for example, one or more sensors on flow modulator 100, e.g., at the sealing zones of upstream component 102 and downstream component 106, imaging guidance such as fluoroscopy, ultrasound for evaluating the diameter change over time, and/or other external transmitters for measuring other derived parameters that may be used to measure the diameter change over time. Additionally, one or more sensors and/or imaging guidance may be used to measure the diameter of the nozzle and/or fluid velocity through the nozzle over time.

As an additional or alternative way to enhance rigidity of the intermediate section of flow modulator 100, the struts of frame 120 at the intermediate section may be wider and/or thicker than the struts of frame 120 at the more flexible portions, as described in U.S. Patent No. 11,324,619. Additionally or alternatively, the lengths of the cells formed by the struts of frame 120 may be shortened and/or the number of cells for a given length of frame 120 may be decreased to increase rigidity. In accordance with another aspect of the present disclosure, the relative flexibility between the portions of the frame may be selected using different shaped cells, e.g., diamond shape or hexagonal shape. In addition, the cells having larger overall void space area may be stronger than the cells having a larger overall working area.

In addition, the downstream end of uncovered portion 132 of flow modulator 100 may include one or more anchors 134 for assisting in maintaining downstream component 106 in its collapsed, delivery state upon exposure from a delivery sheath, as described in U.S. Patent No. 11,324,619. For example, plurality of anchors 134 are configured to be coupled to a delivery device to maintain downstream component 106 in a collapsed delivery state upon exposure to a body lumen from a sheath of the delivery device to facilitate readjustment of flow modulator 100 within the body lumen. In addition, plurality of anchors 134 may function as a downstream component retrieval portion in addition to the retrieval portion of upstream component 102, such that flow modulator 100 may be retrieved from the jugular.

In accordance with one aspect of the present disclosure, the downstream-most portion of downstream component 106, e.g., portion 132, may form an atraumatic end of flow modulator 100 to prevent vessel damage and flare out during device crimping, and to give the distal end integrity. In the expanded, deployed state, the atraumatic end of portion 132 curves inward away from the body vessel inner wall. Accordingly, even after downstream component 106 is in its expanded, deployed state, flow modulator 100 may be readjusted within the body lumen with a reduced risk of injury to the vessel wall of the body lumen due to the distal end of flow modulator 100. In this embodiment, the cells of frame 120 forming portion 132 adjacent to the atraumatic end preferably is uncoated as shown in FIG. 5, such that the fluid stream flows out through exit 107 of downstream component 106 and across the uncoated, bare-metal frame of the atraumatic tip without additional acceleration due to a convergence of the flow path.

Flow modulator 10 further may include one or more various types of fixation elements for securing flow modulator 100 within a vessel, and thereby prevent migration of flow modulator 100 within the vessel, as described in International PCT Patent Application Serial No. PCT/IB2022/060573.

Moreover, the flow modulators described herein may be used in conjunction with an external pump and a control system as described in WO 2020/109979. For example, the external pump may be an intermittent pneumatic compression (IPC) or a cardiac enhanced external counter-pulsation (EECP) pump (such as the ArtAssist® device, available by ACI Medical, San Marcos, California). The pump may be programmed to mimic the natural pumping action of an ambulatory calf and/or foot to move blood in the deep veins of the leg, thereby reducing deep vein thrombosis formation. In addition, the pump may provide power to the flow modulator. The external pump and the control system may be fully mobile and/or battery operated. For example, the external pump and the control system be worn by the patient, e.g., around the patient's leg.

Flow modulator 100 may be deployed and retrieved using a delivery sheath. For example, flow modulator 100 may be fully contained within the delivery sheath prior to placement within the vessel so that flow modulator 100 may be delivered to and deployed at the desired location within the vessel. When the distal end of the delivery sheath is positioned at the desired deployment location, the delivery sheath may be retracted, e.g., pulled in the upstream direction while flow modulator 100 remains stationary relative to the vessel. When removal/retrieval of flow modulator 100 is desired, flow modulator 100 may collapsed towards its collapsed, delivery state, e.g., by pulling retrieval portion 126 via hook 130, and the delivery sheath may be advanced, e.g., pushed in a downstream direction over flow modulator 100, to thereby collapse flow modulator 100 within the delivery sheath for removal. When flow modulator 100 is again collapsed within the delivery sheath, the delivery sheath containing flow modulator 100 may be repositioned within the vessel or removed from the vessel.

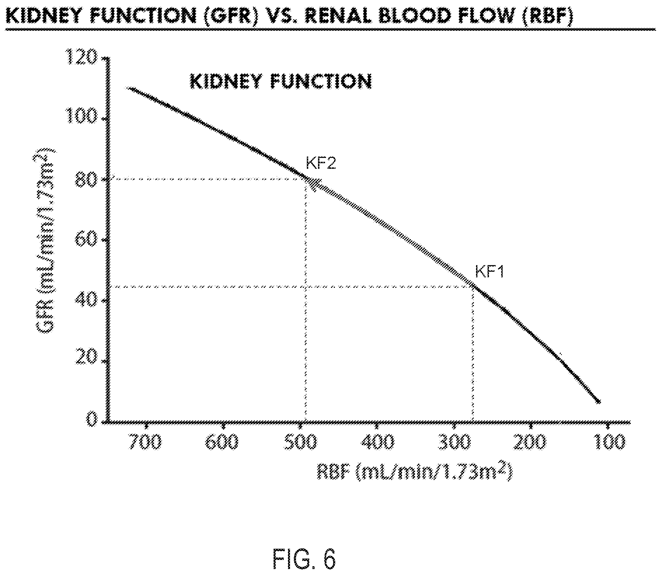

Referring now to FIG. 6, a graph illustrating improvement of kidney function responsive to flow modulator 10 and flow modulator 100 is provided. In FIG. 6, improvement of kidney function resulting from flow modulator 10 is indicated by KF1, and improvement of kidney function resulting from flow modulator 100 is indicated by KF2. As shown in FIG. 6, for flow modulator 10, renal blood flow (RBF) improvement for very low IVC flow rates was limited for GFR of up to approximately 45 mL/min/1.73m$^2$; whereas, for flow modulator 100, at similar IVC flow conditions, RBF improvement was available for patients with GFR of up to approximately 80 mL/min/1.73m$^2$. Accordingly, use of flow modulator 100 may increase the patient population to include patients having GFR of up to at least 80 mL/min/1.73m$^2$.

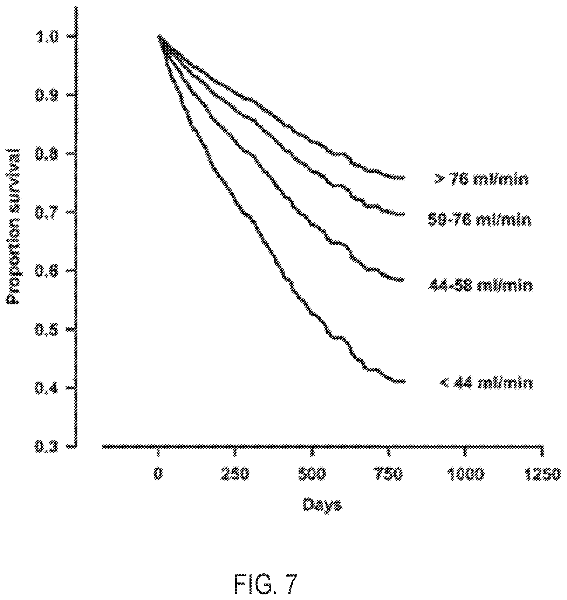

FIG. 7 illustrates a representative study of survival rates for patient populations having various GFRs. Hillege, Hans L., et al. "Renal function, neurohormonal activation, and survival in patients with chronic heart failure." Circulation 102.2 (2000): 203-210. In FIG. 7, a majority of patients had GFR higher than 45 mL/min, e.g., 25% had less than 44 mL/min, 25% had between 44-58 mL/min, 25% had between 59-76 mL/min, and 25% had greater than 76 mL/min. Thus, by increasing the GFR range for which the flow modulators described herein may improve RBF, the patient population will significantly increase as patients with low GFR face higher risk, e.g., lower survival rate, for morbidity and mortality.

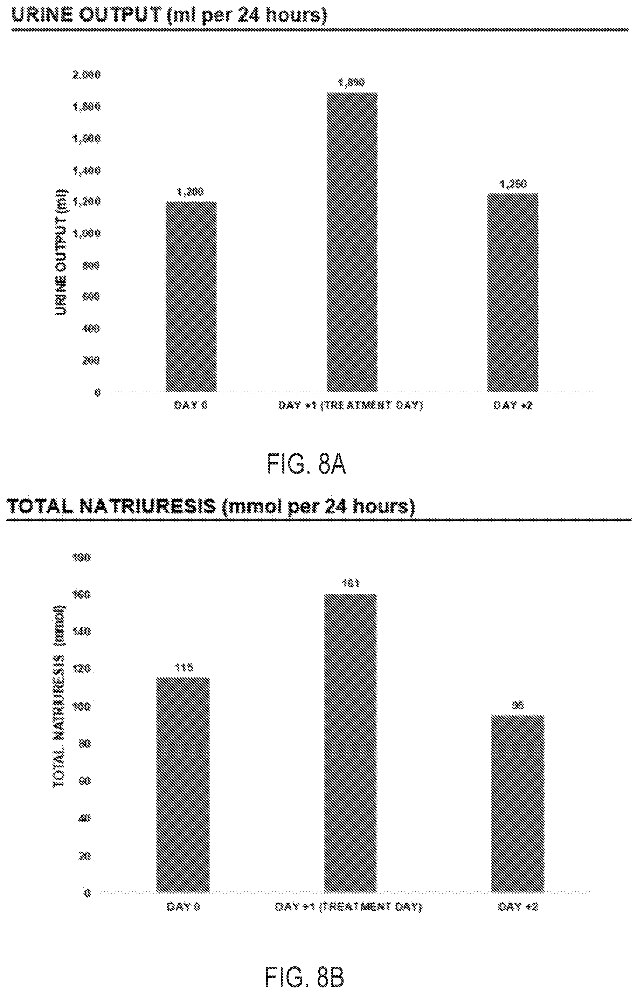

Referring now to FIGS. 8A and 8B, FIG. 8A illustrates a graph of the improvement of urine output responsive to treatment with a flow modulator, such as flow modulator 10 and flow modulator 100. As shown in FIG. 8A, the flow modulator was effective to increase urine output by approximately 690 mL over the course of a 24-hour treatment period. After the flow modulator was removed at the end of the 24-hour treatment period, urine output decreased to approximately the same level as before treatment. FIG. 8B illustrates the improvement of Natriuresis (i.e., sodium excretion by the kidneys) responsive to treatment with a flow modulator over a 24-hour treatment period. As shown in FIG. 8B, Natriuresis increased by approximately 46 mmol during the 24-hour treatment period with the flow modulator. However, after the flow modulator was removed, total sodium weight decreased to approximately 95 mmol, dropping by approximately 56 mmol.

It may be desirable to effectively eliminate the modulation of fluid flow through a body lumen, e.g., the hemodynamic effect of the flow modulators described herein, for example, when the flow modulator is implanted for long term use (e.g., one month, two months, six months, one year, etc.) but only needs to be operable for a predetermined period of time, and wherein the flow modulator remains implanted within the body lumen thereafter, e.g., to avoid having to retrieve the flow modulator. As used herein, the hemodynamic effect of the flow modulator is "effectively eliminated" when the flow modulator is in an inoperable state in the sense that the flow modulator no longer modulates flow through the body lumen as intended, although, in some instances, a minimal amount of hemodynamic effect, e.g., resistance within the body lumen, may still persist by virtue of the flow modulator remaining implanted within the body lumen in the inoperable state.

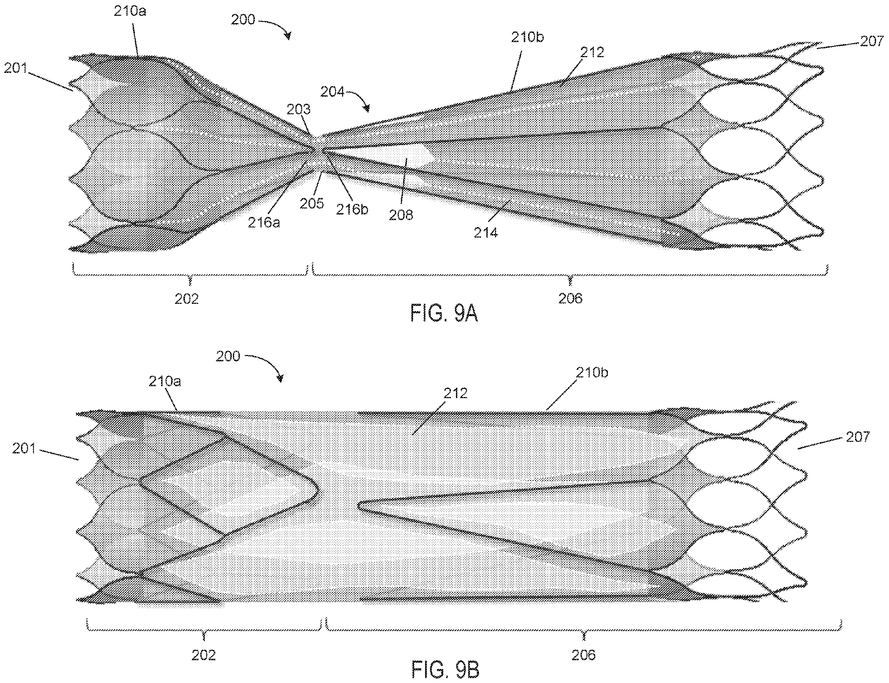

Referring now to FIGS. 9A and 9B, an alternative exemplary flow modulator operative in accordance with the principles of the present disclosure is provided. Flow modulator 200 may be constructed similar to flow modulator 10 and/or flow modulator 100, except that flow modulator 200 may be transitionable between an hourglass configuration for modulating fluid flow therethrough and an expanded, cylindrical configuration suitable for long term implantation when fluid flow modulation may no longer be required, as described in further detail below. For example, as shown in FIG. 9A, flow modulator 200 may have upstream portion 202, downstream portion 206, and entrainment region 204 disposed between upstream portion 202 and downstream portion 206. Like flow modulator 10 and flow modulator 100, flow modulator 200 may be sized and shaped to be implanted in a body lumen, and may be compressed for delivery (e.g., percutaneous delivery with a delivery sheath) and expanded upon deployment (e.g., self-expanding upon release from the end of the delivery sheath or balloon expandable). Moreover, upstream portion 202 has inlet 201 and outlet 203, and has a cross-sectional flow area that converges in a downstream direction, e.g., from upstream portion 202 towards downstream portion 206, along part or all of the length of upstream portion 202, thereby forming a nozzle. In this manner, upstream portion 202 accelerates flow of fluid through upstream portion 202. Downstream portion 206 has entry 205 and exit 207, and has a cross-sectional flow area that diverges in a downstream direction along part or all of the length of downstream portion 206, thereby forming a diffuser.

Entrainment region 204 may include one or more openings 208 designed to entrain fluid into a stream of fluid flowing from upstream portion 202 to downstream portion 206. Specifically, fluid flow through upstream portion 202 to downstream portion 206 creates a lower pressure region in the vicinity of entrainment region 204, which entrains fluid into the stream of fluid flowing across entrainment region 204. Fluid entrainment is induced by shear-induced turbulent flux. In accordance with the principles of the disclosure, such entrainment is expected to transport blood or other body fluids to or from a region so as to improve organ function (e.g., from the renal vein(s) to the inferior vena cava to promote better functionality of the kidney(s) and/or from the hepatic vein(s) to the inferior vena cava to improve liver function, thereby treating disorders and/or diseases such as heart failure).

As shown in FIG. 9A, openings 208 may extend longitudinally along at least a portion of downstream portion 206 (e.g., originating adjacent to the narrowest portion of downstream portion 206, and extending along at least a portion of downstream portion 206). For example, the upstream end of openings 208 may be disposed immediately downstream of outlet 203 of upstream portion 202, e.g., at entry 205 of downstream portion 206. Alternatively, the upstream end of openings 208 may be disposed, e.g., 0-5 mm downstream of outlet 203 of upstream portion 202. Upstream portion 202 may include a distal sealing zone, which contacts the wall of the body lumen in its expanded deployed state, and a portion upstream of the sealing zone, which may adapt to the vessel without damaging the vessel to prevent migration of flow modulator 200 during, e.g., coughing or other events that may cause a dramatic change in vessel diameter. Downstream portion 206 similarly may include a proximal sealing zone, which contacts the wall of the body lumen in its expanded deployed state, and a portion downstream of the sealing zone.

Flow modulator 200 may be constructed as a graft, stent, stent graft, or the like, and is formed of biocompatible materials, such as stainless steel or Nitinol. For example, flow modulator 200 may be formed of one or more wire frames, and a polymeric coating. Unlike flow modulator 10 and flow modulator 100, flow modulator 200 may be configured to transition from an hourglass configuration (e.g., FIG. 9A) to provide fluid modulation through the body lumen, to a cylindrical configuration (e.g., FIG. 9B) where flow modulator 200 expands to fit the internal diameter of the body lumen in which flow modulator 200 is disposed, so as to not obstruct fluid flow through the lumen in which flow modulator 200 is disposed. The cylindrical configuration may eliminate the entrainment associated with the hourglass configuration. This may be advantageous for flow modulators implanted for long term use (e.g., one month, two months, six months, one year, etc.) that are configured to be operable for a predetermined period of time, and remain implanted within the body lumen thereafter, e.g., to avoid having to retrieve the flow modulator or in case the treatment is no longer needed.

For example, as shown in FIGS. 9A and 9B, flow modulator 200 may be formed of first frame portion 210a forming upstream portion 202 and second frame portion 210b forming downstream portion 206, wherein second frame portion 210b is not directly coupled to first frame portion 210a. Accordingly, as shown in FIGS. 9A and 9B, the frame forming flow modulator 200 may include a gap between first frame portion 210a and second frame portion 210b. Polymeric coating 212 may cover the entire flow modulator 200 (except at openings 208), such that polymeric coating 212 extends across the gap between first frame portion 210a and second frame portion 210b, thereby forming flow modulator 200.

For example, first frame portion 210a and second frame portion 210b may be formed in the cylindrical configuration, e.g., thermally shape-set in the cylindrical configuration, and then manipulated into the hourglass configuration where first frame portion 210a forms a nozzle and second frame portion 210b forms a diffuser. Polymeric coating 212 may then be applied to first frame portion 210a and second frame portion 210b in the hourglass configuration, such that flow modulator 200 is maintained in the hourglass configuration. In some embodiments, flow modulator 200 may be maintained in the hourglass configuration via one or more fasteners, e.g., fasteners 216a, 216b, e.g., wires or filaments, disposed around (or within or inside of) polymeric coating 212 at the stent junction at entry 205 of downstream portion 206 and/or at the stent junction at exit 203 of upstream portion 202, as shown in FIG. 9A, such that upon release/breakage of fasteners 216a, 21b, first frame portion 210a and second frame portion 210b may return to the heat-set cylindrical configuration, as shown in FIG. 9B. Fasteners 216a, 216b may be fixed to one or all of the stent junctions in the nozzle portion of flow modulator 200, such that upon release/breakage of fasteners 216a, 216b, e.g., due to balloon inflation, fasteners may remain attached to first frame portion 210a, thereby preventing emboli flow of fasteners 216a, 216b downstream. Alternatively, flow modulator 200 may not require fasteners for maintaining flow modulator 200 in the hourglass configuration, such that polymeric coating 212 alone may be sufficient to maintain flow modulator 200 in the hourglass configuration.

Polymeric coating 212 may also include soft points, e.g., weak line(s) 214, to facilitate expansion of flow modulator 200 to the cylindrical configuration. Because polymeric coating 212 does not have a surface suitable for the cylindrical configuration, weak lines 214 may separate or tear upon expansion into the cylindrical configuration. For example, upon release/breakage of fasteners 216a, 216b, e.g., via cutting, ablation, degradation, balloon expansion within the lumen of flow modulator 200, polymeric coating 212 may tear along weak line(s) 214 to permit flow modulator 200 to transition to the cylindrical configuration. Alternatively, weak lines 214 may be manually separated (e.g., punctured, torn, sliced, etc.) before removing fastener(s) 216a, 216b, such that flow modulator 200 naturally expands to the cylindrical configuration once fastener(s) 216a, 216b are released. The number of weak lines 214 may be selected based on the number of cells of first frame portion 210a and second frame portion 210b in the circumferential direction, and on the frame design. For example, polymeric coating 212 may include one or more weak lines per cell, or alternatively, one weak line every other cell in the circumferential direction.

As the length of flow modulator 200 may change upon the transition from the hourglass configuration to the cylindrical configuration, e.g., due to the increased diameter along the length of flow modulator 200, polymeric coating 200 may split circumferentially to separate first frame portion 210a and second frame portion 210b, such that the separated components are held in position within the body lumen via their respective sealing zones, as described above. Additionally, due to the large tearing of polymeric coating 212 as flow modulator 200 transitions from the hourglass configuration to the cylindrical configuration, the remaining polymeric coating on first frame portion 210a and second frame portion 210b may not block fluid flow entering the body lumen, e.g., the IVC, from the branch vessels, e.g., the renal veins. Accordingly, when flow modulator 200 is implanted in the IVC in the cylindrical configuration, most of a renal ostia will be unblocked by the remaining polymeric coating on the frames of flow modulator 200.

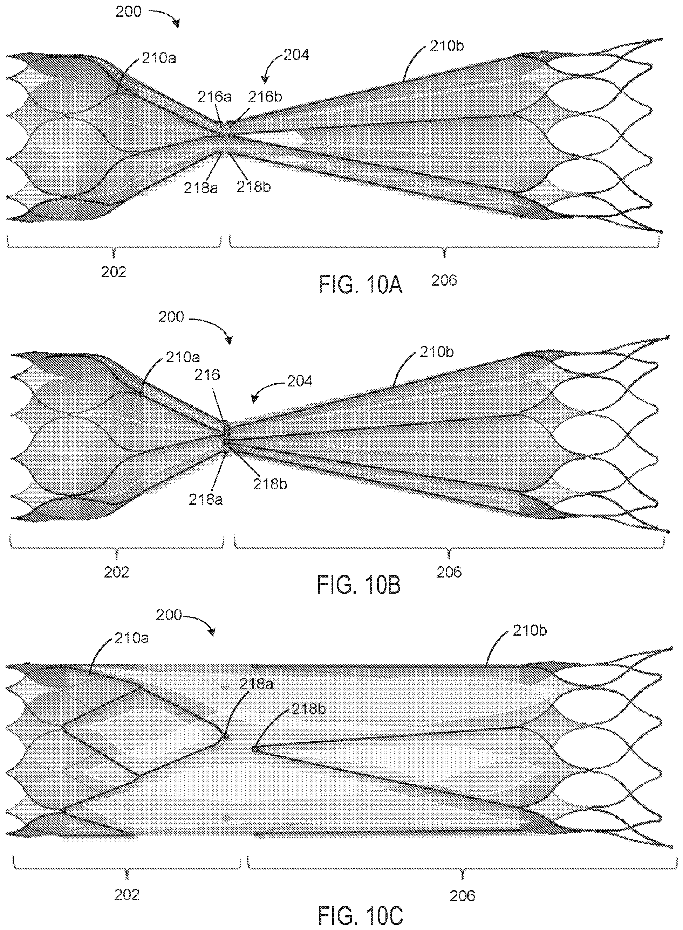

Referring now to FIGS. 10A to 10C, first frame portion 210a and/or second frame portion 210b may further include one or more rings configured to receive and secure one or more fasteners to maintain flow modulator 200 in the hourglass configuration. For example, a downstream end of first frame portion 210a may include a first set of rings 218a, and an upstream end of second frame portion 210b may include a second set of rings 218b, as shown in FIG. 10A. Accordingly, rings 218a and rings 218b may be configured to receive and secure fastener 216a and fastener 216b, respectively. As shown in FIG. 10B, a single fastener 216 may be used to secure first frame portion 210a to second frame portion 210b via rings 218a and rings 218b, such that the gap between first frame portion 210a and second frame portion 210b is reduced or otherwise eliminated in the hourglass configuration. Accordingly, when fastener 216 is released/broken, flow modulator 200 may transition from the hourglass configuration to the cylindrical configuration, as shown in FIG. 10C.

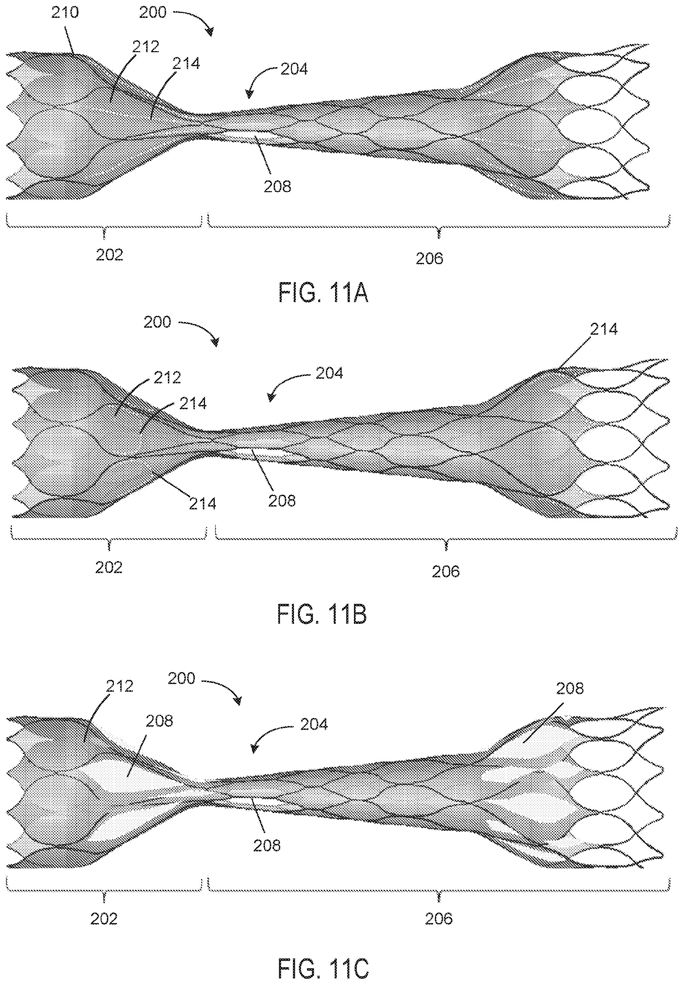

Referring now to FIGS. 11A to 11C, the hemodynamic effect of the implanted flow modulator may be effectively eliminated for a flow modulator formed of a single frame. For example, flow modulator 200 may be formed of a single frame, e.g., frame 210. As shown in FIG. 11A, polymeric coating 212 may include one or more weak lines 214 extending along a length of upstream region 202 and/or downstream region 206 of flow modulator 200, such that polymeric coating 212 may tear along weak lines 214 to create a flap opening via an application of force, as described above, while retaining the hourglass configuration of flow modulator 200. Weak lines 214 may be formed of a plurality of micro holes in polymeric coating 212. Additionally, or alternatively, as shown in FIG. 11B, polymeric coating 212 may include one or more weak lines 214 extending within one or more cells of upstream region 202 and/or downstream region 206 of flow modulator 200, such that polymeric coating 212 may tear along weak lines 214 within the one or more cells to create flap openings to effectively eliminate the hemodynamic effect.

As shown in FIG. 11C, flow modulator 200 may include additional openings 208 within polymeric coating 212 along either or both of upstream portion 202 and downstream portion 206. For example, the holes/openings may be creating via a cutting or puncture tool, or alternatively, a power-driven tool via, e.g., heat, RF energy, etc. Presence of additional openings may effectively eliminate the hemodynamic effect, and accordingly, entrainment within flow modulator 200, such that flow modulator 200 is functional as only a stent, or stent-like device.

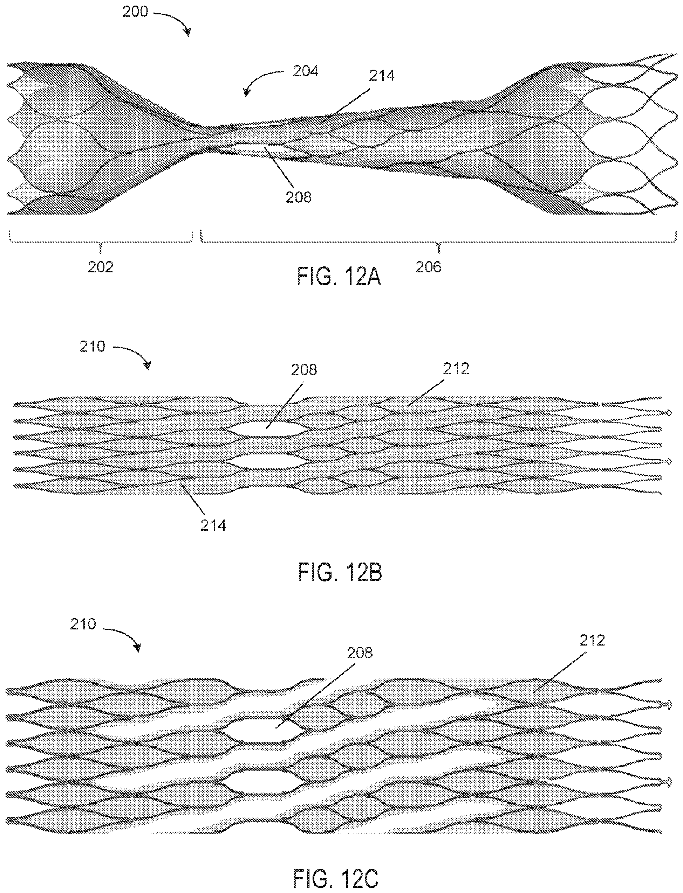

Referring now to FIGS. 12A to 12C, flow modulator 200 may be twisted between the sealing zones at the upstream and downstream ends of flow modulator 200. The twisted flow modulator 200, shown in FIG. 12A, may permit easier expansion of flow modulator 200 from the hourglass configuration to the cylindrical configuration. As shown in FIG. 12B, weak line(s) 214 may be formed in the center of flow modulator 200 along the twist. For example, weak line(s) 214 may be disposed on polymeric coating 212 within one or more cells extending continuously along the twist between the sealing zones. Upon transitioning from the hourglass configuration to the cylindrical configuration, polymeric coating 212 may separate along weak line(s) 214 to compensate for the increased surface area of polymeric coating 212, as shown in FIG. 12C.

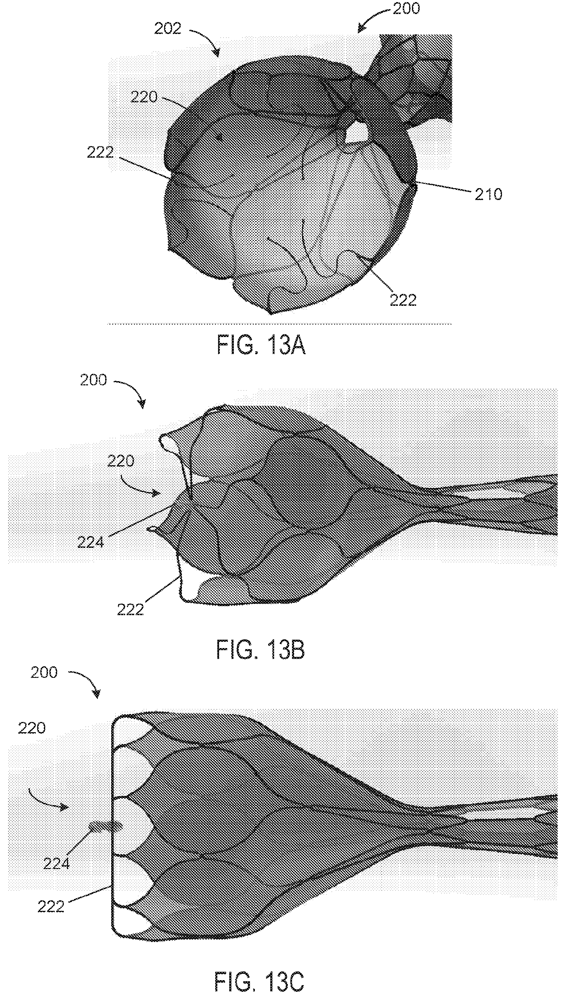

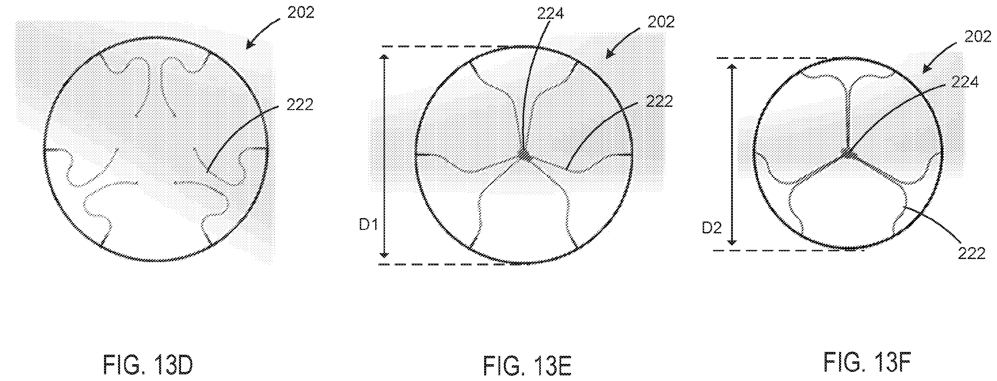

Referring now to FIGS. 13A to 13F, flow modulator 200 may also include a hook assembly 220 affixed to upstream portion 202 and/or downstream portion 206 to facilitate placement and/or removal of flow modulator 200. As shown in FIGS. 13A and 13D, when flow modulator 200 is formed, radial connectors 222 may extend from frame 210, such that the distal ends of radial connectors 222 are unconnected. As shown in FIG. 13A, the hooked ends of radial connectors 222 may not protrude, or only minimally protrudes, from the sealing zones of flow modulator 200. Radial connectors 222 may then be secured to hook 224, while in tension, to form hook assembly 220, as shown in FIGS. 13B and 13C. Alternatively, in some embodiments, the hooked ends of radial connectors may at least partially protrude in a downstream direction within flow modulator 200, such that when coupled to hook 224, hook 224 may be positioned downstream of the sealing zone at the upstream end of flow modulator 200. Similarly, when radial connectors 222 extend from the downstream end of flow modulator 200, they may at least partially protrude in an upstream direction such that hook 224 may be positioned upstream of the sealing zone at the downstream end of flow modulator 200. The radial stiffness of the sealing zone may be much greater than that of radial connectors 222, and thus, upon assembly of hook 224, the outer diameter of flow modulator 200 may not be significantly altered due to tension loads by radial connectors 222. Accordingly, radial connectors 222 may function like a spring such that hook assembly 220 does not otherwise alter the structure of flow modulator 200, e.g., responsive to changes in the sealing zone diameter. In this configuration, any reduction of the sealing zone diameter (e.g., from deployment in oversized IVC) will not elongate the hook location in relation to the sealing zone and the hook effective length will remain constant. For example, the sealing zone of upstream portion 202 having hook assembly 220 may be reduced from an initial diameter D1 (FIG. 13E) to a second diameter D1 (FIG. 13F), such that radial connectors 222 compress in a radially direction inward, such that hook 224 remains within the plane of the sealing zone.

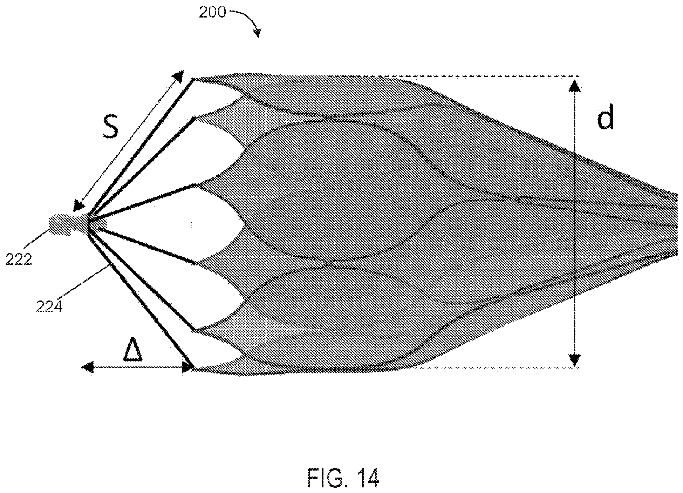

Alternatively, in some embodiments, radial connectors 222 may have a fixed length, as shown in FIG. 14.

Accordingly, additional tension placed on radial connectors 222 having a fixed length may result in elongation of flow modulator 200. This elongation is represented by the following equation:

$$\Delta = \sqrt{\left(\frac{D}{2}\right)^2 - \left(\frac{d}{2}\right)^2}.$$

To prevent elongation, radial connectors 222 may change length under tension. For example, radial connectors 222 may lengthen under tension, and shorten when tension is released. This is necessary to maintain the position of flow modulator 200 within the body lumen, such that the sealing zone(s) remain in contact with the interior surface of the lumen.

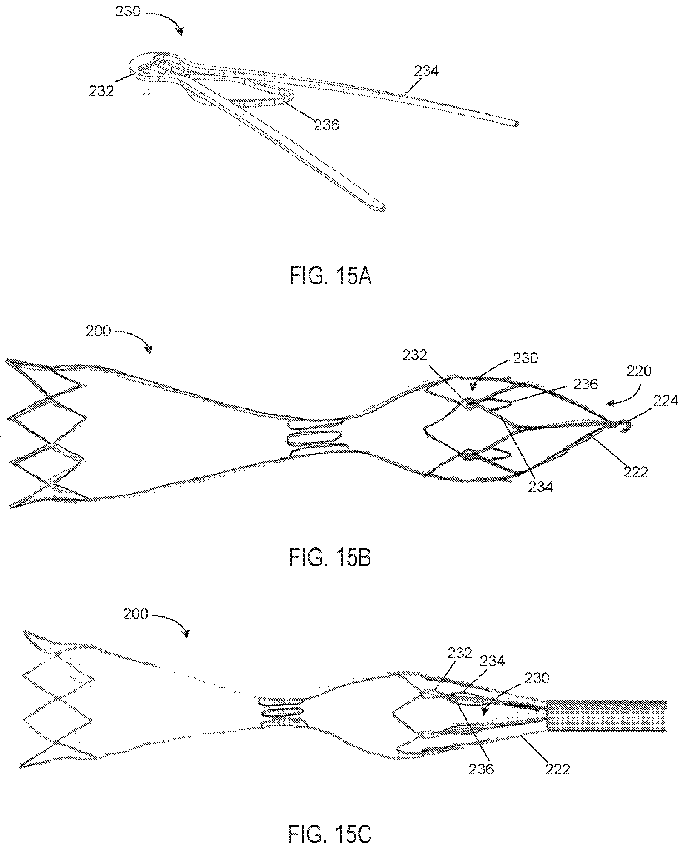

Referring now to FIGS. 15A to 15C, flow modulator 200 may also include an anchor 230. As shown in FIG. 15A, anchor 230 may be integrally formed and may include frame portions 232, strut portions 234, and a loop portion 236. Frame portions 232 may be attached to the end points of frame 210. As shown in FIG. 15B, when anchor(s) 230 are attached to frame 210, strut portions 234 of each anchor 230 extend towards and connect with one another, such that all anchors 230 form a single integral structure. Frame portion(s) 232 are effective to secure anchor(s) 230 to frame 210, without placing frame 210 under tension. Prior to retrieval, loop portion(s) 236 extend away from frame 210 towards the retrieval end. When tension is applied to hook assembly 220 to retrieve flow modulator 200, as described above, strut portions 234 of each anchor 230 may move toward each other, as shown in FIG. 15C, thus lowering loop portion 236 towards the retrieval sheath centerline and preventing it from interfering with the shaft tip, such that flow modulator 200 may be continuously inserted into the retrieval sheath.

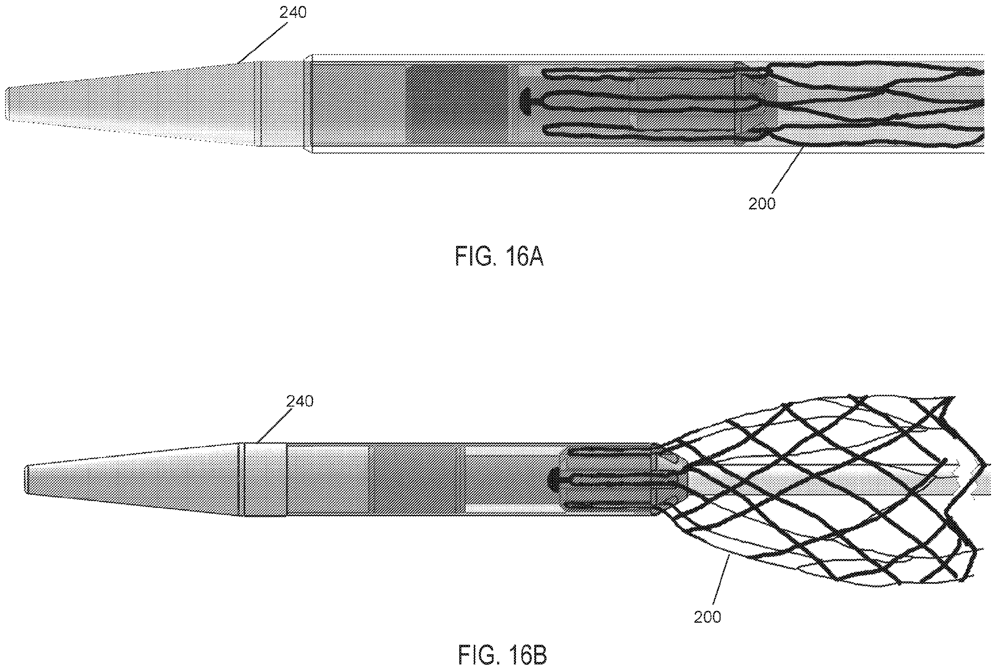

Referring now to FIGS. 16A and 16B, delivery sheath 240 may be provided for deploying flow modulator 200. Flow modulator 200 may be stored within delivery sheath 240 during delivery. Delivery sheath 240 may be configured to permit shortening of flow modulator 200, such that flow modulator 200 may be partially released from delivery sheath 240 to a partially-expanded state, which may enable more uniform release without sudden and/or uncontrolled expansion during deployment of flow modulator 200.

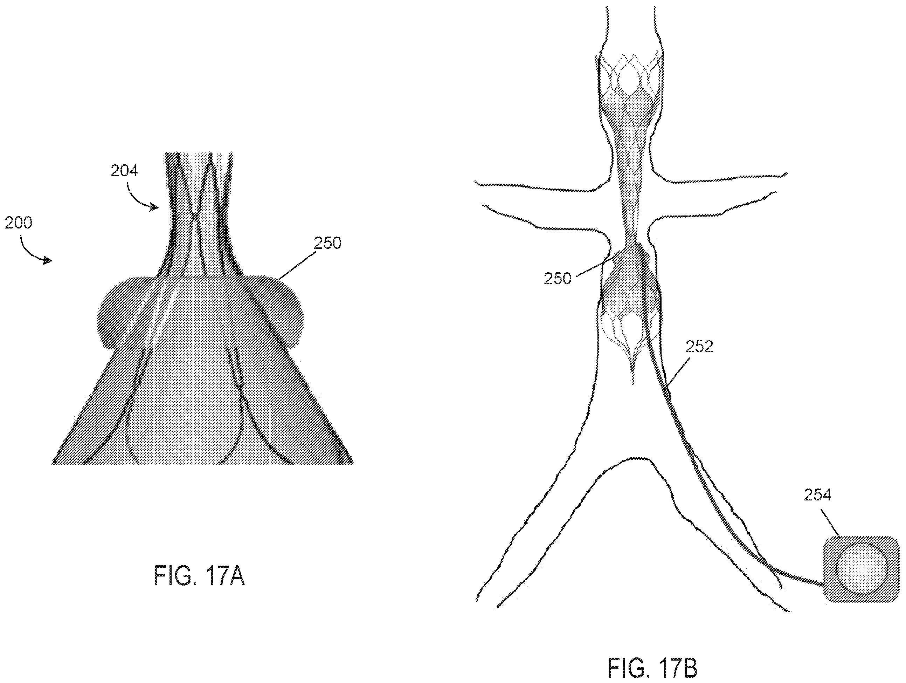

Referring now to FIGS. 17A and 17B, the diameter of the nozzle of flow modulator 200 may be adjusted *in vivo*. As shown in FIG. 17A, annular balloon 250 may be disposed around the circumference of the nozzle of flow modulator 200, e.g., adjacent to the outlet of the nozzle, such that inflation of balloon 250 radially compresses the outlet of the nozzle and decreases the internal diameter thereof. As shown in FIG. 17B, balloon 250 may be fluidically coupled to access point 254, e.g., a subdermal port, via inflation line 252 for inflation/deflation of balloon 250. Accordingly, fluid may be introduced to balloon 250 via access point 254. As will be understood by a person having ordinary skill in the art, other known techniques for adjusting shunt size may be used to adjust the diameter of the nozzle size *in vivo.*

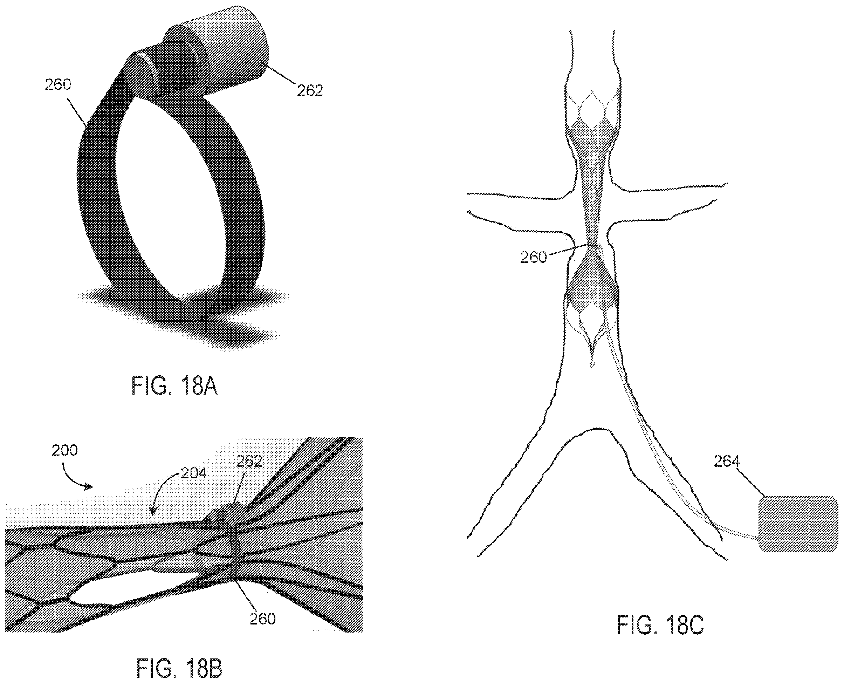

Referring now to FIGS. 18A to 18C, instead of an annular balloon, flow modulator 200 may include band 260 for adjusting the diameter of the outlet of the nozzle *in vivo.* Band 260 may include motor 262 disposed thereon. Motor 262 may be wrapped around at least a portion of band 260, such that motor 262 may be actuated to increase or reduce the diameter of band 260, to thereby expand or contract band 260. As shown in FIG. 18B, band 260 may be disposed around the circumference of flow modulator 200 adjacent to the outlet of the nozzle. Band 260 may be configured to expand or contract based on the flow data sensed by one or more sensors upstream and/or downstream of flow modulator 200. For example, as shown in FIG. 18C, the circumference of band 260 may selectively adjusted via external control unit 264 operatively coupled to motor 262 of band 260.

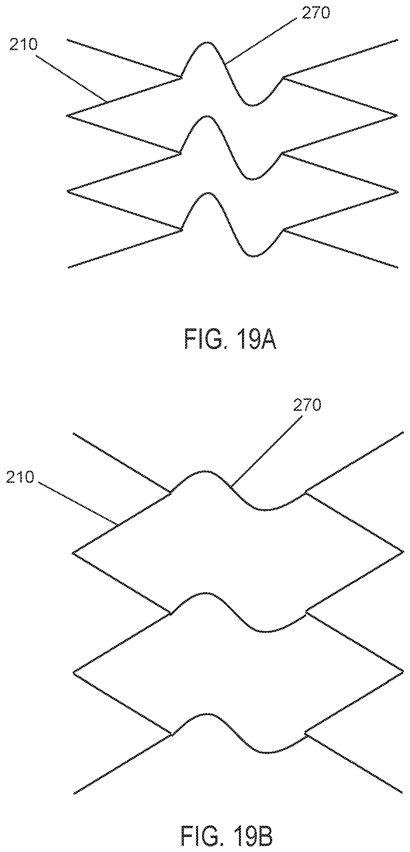

Referring now to FIGS. 19A and 19B, the frame of flow modulator 200 may include connectors 270 disposed at one or more stent junctions to facilitate expansion of flow modulator 200 from the hourglass configuration to the cylindrical configuration, as described above. FIG. 19A shows connectors 270 in a relaxed configuration, whereas FIG. 19B shows connectors 270 in an expanded configuration. Accordingly, a segment of the struts adjacent to connectors 270 may maintain its overall length as flow modulator 200 transitions from the hourglass configuration to the cylindrical configuration. Connectors 270 may also minimize tension and/or compression forces on, for example, a blood vessel wall as a result of contact of sealing zone(s) of flow modulator 200.

The flow modulators described herein may be installed in the inferior vena cava at the branch to a hepatic vein. Accordingly, additional blood may be entrained from the hepatic veins into the IVC, thereby improving splanchnic circulation. Acutely or chronically implanting a flow modulator in the IVC adjacent the hepatic veins may improve liver function and/or may be used instead of, or in parallel to, a TIPS procedure. Advantageously, the flow modulator improves hepatic flow to the inferior vena cava allowing blood to enter the liver for natural filtering (in contrast to a TIPS procedure that bypasses blood from the liver). The flow modulator, whether used together with a TIPS procedure or in place of a TIPS procedure, is expected to treat conditions such as portal hypertension (often due to liver cirrhosis) which frequently leads to intestinal bleeding, life-threatening esophageal bleeding (esophageal varices), the buildup of fluid within the abdomen (ascites), and/or hepatorenal syndrome.

Additionally or alternatively, the flow modulators described herein may be installed in the inferior vena cava to entrain additional blood from both the renal and hepatic veins. For example, the exit of the downstream component may be downstream to the hepatic vein while the inlet of the upstream component is upstream to the renal veins. In one study, the mean distance from a downstream renal vein to the hepatic vein was 6 cm, and the mean distance from the upstream-most renal vein to the downstream-most renal vein was 2.5 cm, and thus a flow modulator having an overall distance of 8.5 cm between the fixation areas of upstream component 102 and downstream component 106 may be anchored within the IVC to improve both renal and hepatic perfusion simultaneously.

Moreover, the flow modulators described herein may be installed in an aneurysm to lower pressure at the aneurysm site, and reduce the risk that the aneurysm will increase in size or burst, and may even cause the aneurysm to decrease in size. In this case, the flow modulator is expected to provide beneficial effect even without sealing against the aneurysm. In addition, if there are one or more side branch lumens at or near the aneurysm site, the device not only will reduce the pressure but also permit blood to flow to the side branches. In this application, the device of the present invention provides significant benefit as compared to previously-known circular stent grafts, which disadvantageously may block the side branches. If there are no side branches, then the device is expected to reduce pressure without increasing the blood flow. Optionally, a filter may be used with the flow modulator to prevent embolic debris from flowing from the aneurysm to other blood vessels.

Any of the foregoing embodiments of the device of the present invention may serve to divert emboli or other debris, so there is no need to use an extra filtration device. One example is using the upstream component or downstream component at or near the carotid arteries to divert emboli or other debris.

## Claims

1. A flow modulator device (100) for altering fluid flow through a body lumen, the body lumen coupled to a branch lumen, the flow modulator device (100) comprising:

   an upstream component (102) having an inlet (101), an outlet (103), and a cross-sectional flow area that converges from the inlet (101) towards the outlet (103) to form a nozzle;
   a downstream component (106) having an entry (105), an exit (107), and a cross-sectional flow area that diverges from the entry (105) towards the exit (107) to form a diffuser, the downstream component (106) comprising a first diverging portion (106a) and a second diverging portion (106b) downstream from the first diverging portion (106a), the first diverging portion's average angle of divergence greater than the second diverging portion's average angle of divergence; and

an entrainment region (104) between the inlet (101) of the upstream component (102) and the exit (107) of the downstream component (106), the entrainment region (104) comprising one or more openings (108) extending across at least a portion of both the first and second diverging portions (106a, 106b) of the downstream component (106);

wherein the flow modulator device (100) is configured to be positioned within the body lumen such that the nozzle accelerates a fluid stream passing through the upstream component (102) towards the downstream component (106) to generate a low pressure region in a vicinity of the entrainment region (104) that entrains additional fluid into the fluid stream via the one or more openings (108) as the fluid stream passes into the downstream component (106).

2. The flow modulator device (100) of claim 1, wherein the upstream component (102) and the downstream component (106) are formed from a frame (120), and wherein the flow modulator device (100) comprises a coating (122) on at least a portion of the upstream component (102) and the downstream component (106), the one or more openings (108) defined by one or more uncoated portions of the frame,

and preferably
wherein the frame (120) defines a plurality of cells.

3. The flow modulator device (100) of claim 2, wherein at least a portion (106d) of a distal portion of downstream component (106) is uncoated, the distal portion configured to adapt with the body lumen to thereby prevent migration of the flow modulator device (100) within the body lumen.

4. The flow modulator device (100) of any one of the preceding claims, wherein the one or more openings (108) comprises a plurality of openings circumferentially spaced around the entrainment region (104).

5. The flow modulator device (100) of any one of the preceding claims, wherein the downstream component (106) comprises a third diverging portion (106c) downstream from the second diverging portion (106b), the third diverging portion's average angle of divergence greater than the second diverging portion's average angle of divergence.

6. The flow modulator device (100) of any one of the preceding claims, wherein the flow modulator device (100) is configured to transition from a collapsed delivery state to an expanded deployed state within the body lumen,

and preferably
wherein, in the expanded deployed state, a proximal portion of the upstream component (102) and a distal portion of the downstream component (106) are configured to adapt with the body lumen.

7. The flow modulator device (200) of claim 6, wherein the upstream component (202) and the downstream component (206) are formed from a frame (210), the flow modulator device (200) comprising an anchor (230) comprising:

a frame portion (232) configured to be coupled to the frame (210);
a pair of struts (234) comprising a downstream portion extending from the frame portion (232) and away from each other in an upstream direction, and an upstream portion extending from the downstream portion and toward each other in the upstream direction; and
a loop portion (236) extending axially and radially outward from the frame portion (232) in the upstream direction between the pair of struts (234), the loop portion (236) configured engage the body lumen in the expanded deployed state to secure the flow modulator device (200) within the body lumen,
wherein, upon transitioning of the flow modulator device (200) from the expanded deployed state to the collapsed delivery state, the pair of struts (234) are configured to move towards each other to cause the loop portion (236) to contract radially inward, such that the loop portion (236) does not extend radially beyond the pair of struts (234).

8. The flow modulator device (100) of any one of the preceding claims, further comprising a retrieval portion (126) extending from the inlet (101) of the upstream component (102) and converging in an upstream direction toward an upstream end of the flow modulator device (100), the retrieval portion (126) configured to facilitate retrieval of the flow modulator device (100),

and preferably
wherein the retrieval portion (126) comprises a hook (130) at the upstream end of the flow modulator device (100), the hook (130) configured to be pulled to collapse the upstream component (102).

9. The flow modulator device (100) of any one of the preceding claims, wherein the diffuser decelerates the fluid stream having the entrained additional fluid passing through the downstream component (106).

**10.** The flow modulator device (100) of any one of the preceding claims, wherein the downstream component's length is greater than the upstream component's length.

**11.** The flow modulator device (100) of any one of the preceding claims, wherein the upstream component's average angle of convergence is greater than the downstream component's average angle of divergence.

**12.** The flow modulator device (100) of any one of the preceding claims, wherein the one or more openings (108) extend into the flow modulator device's narrowest section.

**13.** The flow modulator device (200) of any one of the preceding claims, further comprising an adjustor (250, 260) disposed around a circumference of the upstream component (202) adjacent the outlet (203) of the upstream component (202), the adjustor (250, 260) configured to be actuated to adjust an internal diameter of the outlet (203) *in vivo*.

**14.** The flow modulator device (200) of claim 13, wherein the adjustor (250) comprises:

an annular balloon (250) disposed around the circumference of the upstream component (202) adjacent the outlet (203) of the upstream component (202); and
an access port (254) fluidically coupled to the annular balloon (250) via an inflation line (252), the access port (254) configured to receive and expel fluid to thereby inflate and deflate the annular balloon (250) to adjust the internal diameter of the outlet (203) *in vivo*.

**15.** The flow modulator device (200) of claim 13, wherein the adjustor (260) comprises:

a band (260) disposed around the circumference of the upstream component (202) adjacent the outlet (203) of the upstream component (202);
a motor (262) operatively coupled to the band (260); and
an external control unit (264) operatively coupled to the motor (262), the external control unit (264) configured to actuate the motor (262) to adjust a diameter of the band (260) to adjust the internal diameter of the outlet (203) *in vivo*.

**Patentansprüche**

**1.** Flussmodulatorvorrichtung (100) zum Verändern eines Fluidflusses durch ein Körperlumen, wobei das Körperlumen mit einem Zweiglumen gekoppelt ist, die Flussmodulatorvorrichtung (100) aufweisend:

eine stromaufwärts gelegene Komponente (102), die einen Einlass (101), einen Auslass (103) und eine Querschnittsflussfläche, die von dem Einlass (101) zu dem Auslass (103) hin konvergiert, um eine Düse auszubilden, aufweist;
eine stromabwärts gelegene Komponente (106), die einen Eingang (105), einen Ausgang (107) und eine Querschnittsflussfläche, die von dem Eingang (105) zu dem Ausgang (107) hin divergiert, um einen Diffusor auszubilden, aufweist, die stromabwärts gelegene Komponente (106) aufweisend einen ersten divergierenden Abschnitt (106a) und einen zweiten divergierenden Abschnitt (106b) stromabwärts von dem ersten divergierenden Abschnitt (106a), wobei der durchschnittliche Divergenzwinkel des ersten divergierenden Abschnitts größer als der durchschnittliche Divergenzwinkel des zweiten divergierenden Abschnitts ist; und
eine Mitreißregion (104) zwischen dem Einlass (101) der stromaufwärts gelegenen Komponente (102) und dem Ausgang (107) der stromabwärts gelegenen Komponente (106), die Mitreißregion (104) aufweisend eine oder mehrere Öffnungen (108), die sich über mindestens einen Abschnitt sowohl des ersten als auch des zweiten divergierenden Abschnitts (106a, 106b) der stromabwärts gelegenen Komponente (106) erstrecken;
wobei die Flussmodulatorvorrichtung (100) konfiguriert ist, um derart innerhalb des Körperlumens positioniert zu werden, dass die Düse einen Fluidstrom beschleunigt, der durch die stromaufwärts gelegene Komponente (102) zu der stromabwärts gelegenen Komponente (106) hin hindurchtritt, um eine Niederdruckregion in einer Nähe der Mitreißregion (104) zu erzeugen, die zusätzliches Fluid in den Fluidstrom über die eine oder die mehreren Öffnungen (108) mitreißt, wenn der Fluidstrom in die stromabwärts gelegene Komponente (106) eintritt.

**2.** Flussmodulatorvorrichtung (100) nach Anspruch 1, wobei die stromaufwärts gelegene Komponente (102) und die stromabwärts gelegene Komponente (106) aus einem Rahmen (120) ausgebildet sind und wobei die Flussmodulatorvorrichtung (100) eine Beschichtung (122) auf mindestens einem Abschnitt der stromaufwärts gelegenen Komponente (102) und der stromabwärts gelegenen Komponente (106) aufweist, wobei die eine oder die mehreren Öffnungen (108) durch einen oder mehrere unbeschichtete Abschnitte des Rahmens definiert sind,

und vorzugsweise
wobei der Rahmen (120) mehrere Zellen definiert.

3. Flussmodulatorvorrichtung (100) nach Anspruch 2, wobei mindestens ein Abschnitt (106d) eines distalen Abschnitts der stromabwärts gelegenen Komponente (106) unbeschichtet ist, der distale Abschnitt konfiguriert ist, um sich an das Körperlumen anzupassen, um dadurch eine Migration der Flussmodulatorvorrichtung (100) innerhalb des Körperlumens zu verhindern.

4. Flussmodulatorvorrichtung (100) nach einem der vorstehenden Ansprüche, wobei die eine oder die mehreren Öffnungen (108) mehrere Öffnungen aufweisen, die um die Mitreißregion (104) herum umlaufend beabstandet sind.

5. Flussmodulatorvorrichtung (100) nach einem der vorstehenden Ansprüche, wobei die stromabwärts gelegene Komponente (106) einen dritten divergierenden Abschnitt (106c) stromabwärts von dem zweiten divergierenden Abschnitt (106b) aufweist, wobei der durchschnittliche Divergenzwinkel des dritten divergierenden Abschnitts größer als der durchschnittliche Divergenzwinkel des zweiten divergierenden Abschnitts ist.

6. Flussmodulatorvorrichtung (100) nach einem der vorstehenden Ansprüche, wobei die Flussmodulatorvorrichtung (100) konfiguriert ist, um von einem zusammengeklappten Zuführzustand in einen erweiterten entfalteten Zustand innerhalb des Körperlumens überzugehen,

und vorzugsweise
wobei, in dem erweiterten entfalteten Zustand, ein proximaler Abschnitt der stromaufwärts gelegenen Komponente (102) und ein distaler Abschnitt der stromabwärts gelegenen Komponente (106) konfiguriert sind, um sich an das Körperlumen anzupassen.

7. Flussmodulatorvorrichtung (200) nach Anspruch 6, wobei die stromaufwärts gelegene Komponente (202) und die stromabwärts gelegene Komponente (206) aus einem Rahmen (210) ausgebildet sind, die Flussmodulatorvorrichtung (200) aufweisend einen Anker (230), aufweisend:

einen Rahmenabschnitt (232), der konfiguriert ist, um mit dem Rahmen (210) gekoppelt zu werden;
ein Paar von Streben (234), aufweisend einen stromabwärts gelegenen Abschnitt, der sich von dem Rahmenabschnitt (232) und in einer Stromaufwärtsrichtung voneinander weg erstreckt, und einen stromaufwärts gelegenen Abschnitt, der sich von dem stromabwärts gelegenen Abschnitt und in der Stromaufwärtsrichtung zueinander hin erstreckt; und
einen Schlaufenabschnitt (236), der sich von dem Rahmenabschnitt (232) in der Stromaufwärtsrichtung zwischen dem Paar von Streben (234) axial und radial nach außen erstreckt, wobei der Schlaufenabschnitt (236) konfiguriert ist, um das Körperlumen in dem erweiterten entfalteten Zustand in Eingriff zu nehmen, um die Flussmodulatorvorrichtung (200) innerhalb des Körperlumens zu sichern,
wobei, bei dem Übergehen der Flussmodulatorvorrichtung (200) von dem erweiterten entfalteten Zustand in den zusammengeklappten Zuführzustand, das Paar von Streben (234) konfiguriert ist, um sich zueinander hin zu bewegen, um den Schlaufenabschnitt (236) zu veranlassen, sich derart radial nach innen zusammenziehen, dass sich der Schlaufenabschnitt (236) nicht über das Paar von Streben (234) hinaus radial erstreckt.

8. Flussmodulatorvorrichtung (100) nach einem der vorstehenden Ansprüche, ferner aufweisend einen Rückholabschnitt (126), der sich von dem Einlass (101) der stromaufwärts gelegenen Komponente (102) erstreckt und in einer Stromaufwärtsrichtung zu einem stromaufwärts gelegenen Ende der Flussmodulatorvorrichtung (100) hin konvergiert, wobei der Rückholabschnitt (126) konfiguriert ist, um ein Zurückholen der Flussmodulatorvorrichtung (100) zu ermöglichen,

und vorzugsweise
wobei der Rückholabschnitt (126) einen Haken (130) an dem stromaufwärts gelegenen Ende der Flussmodulatorvorrichtung (100) aufweist, wobei der Haken (130) konfiguriert ist, um gezogen zu werden, um die stromaufwärts gelegene Komponente (102) zusammenzuklappen.

9. Flussmodulatorvorrichtung (100) nach einem der vorstehenden Ansprüche, wobei der Diffusor den Fluidstrom verlangsamt, der das mitgerissene zusätzliche Fluid aufweist, der durch die stromabwärts gelegene Komponente (106) durchtritt.

10. Flussmodulatorvorrichtung (100) nach einem der vorstehenden Ansprüche, wobei die Länge der stromabwärts gelegenen Komponente größer als die Länge der stromaufwärts gelegenen Komponente ist.

11. Flussmodulatorvorrichtung (100) nach einem der vorstehenden Ansprüche, wobei der durchschnittliche Konvergenzwinkel der stromaufwärts gelegenen Komponente größer als der durchschnittliche Divergenzwinkel der stromabwärts gelegenen Komponente ist.

**12.** Flussmodulatorvorrichtung (100) nach einem der vorstehenden Ansprüche, wobei sich die eine oder die mehreren Öffnungen (108) in den engsten Bereich der Flussmodulatorvorrichtung erstrecken.

**13.** Flussmodulatorvorrichtung (200) nach einem der vorstehenden Ansprüche, ferner aufweisend einen Justierer (250, 260), der um einen Umfang der stromaufwärts gelegenen Komponente (202) angrenzend an den Auslass (203) der stromaufwärts gelegenen Komponente (202) angeordnet ist, wobei der Justierer (250, 260) konfiguriert ist, um betätigt zu werden, um einen Innendurchmesser des Auslasses (203) *in vivo* zu justieren.

**14.** Flussmodulatorvorrichtung (200) nach Anspruch 13, wobei der Justierer (250) aufweist:

einen ringförmigen Ballon (250), der um den Umfang der stromaufwärts gelegenen Komponente (202) angrenzend an den Auslass (203) der stromaufwärts gelegenen Komponente (202) angeordnet ist; und
einen Zugangsanschluss (254), der mit dem ringförmigen Ballon (250) über eine Aufblasleitung (252) fluidisch gekoppelt ist, wobei der Zugangsanschluss (254) konfiguriert ist, um Fluid aufzunehmen und auszustoßen, um dadurch den ringförmigen Ballon (250) aufzublasen und zu entleeren, um den Innendurchmesser des Auslasses (203) *in vivo* zu justieren.

**15.** Flussmodulatorvorrichtung (200) nach Anspruch 13, wobei der Justierer (260) aufweist:

ein Band (260), das um den Umfang der stromaufwärts gelegenen Komponente (202) angrenzend an den Auslass (203) der stromaufwärts gelegenen Komponente (202) angeordnet ist;
einen Motor (262), der mit dem Band (260) wirkverbunden ist; und
eine externe Steuereinheit (264), die mit dem Motor (262) wirkverbunden ist, wobei die externe Steuereinheit (264) konfiguriert ist, um den Motor (262) zu betätigen, um einen Durchmesser des Bandes (260) zu justieren, um den Innendurchmesser des Auslasses (203) *in vivo* zu justieren.

**Revendications**

**1.** Dispositif modulateur d'écoulement (100) permettant de modifier un écoulement de fluide à travers une lumière de corps, la lumière de corps étant accouplée à une lumière de branche, le dispositif modulateur d'écoulement (100) comprenant :

un composant amont (102) ayant une entrée (101), une sortie (103) et une zone d'écoulement transversale qui converge de l'entrée (101) vers la sortie (103) pour former une buse ;
un composant aval (106) ayant une entrée (105), une sortie (107) et une zone d'écoulement en section transversale qui diverge de l'entrée (105) vers la sortie (107) pour former un diffuseur, le composant aval (106) comprenant une première partie divergente (106a) et une deuxième partie divergente (106b) en aval de la première partie divergente (106a), l'angle de divergence moyen de la première partie divergente étant supérieur à l'angle de divergence moyen de la deuxième partie divergente ; et
une région d'entraînement (104) entre l'entrée (101) du composant amont (102) et la sortie (107) du composant aval (106), la région d'entraînement (104) comprenant une ou plusieurs ouvertures (108) s'étendant à travers au moins une partie à la fois de la première et de la seconde partie divergente (106a, 106b) du composant aval (106) ;
dans lequel le dispositif modulateur d'écoulement (100) est conçu pour être positionné à l'intérieur de la lumière de corps de telle sorte que la buse accélère un écoulement de fluide passant à travers le composant amont (102) vers le composant aval (106) afin de générer une région de basse pression au voisinage de la région d'entraînement (104) qui entraîne du fluide supplémentaire dans l'écoulement de fluide par l'intermédiaire de la ou des ouvertures (108) lorsque l'écoulement de fluide passe dans le composant aval (106).

**2.** Dispositif modulateur d'écoulement (100) selon la revendication 1, dans lequel le composant amont (102) et le composant aval (106) sont formés à partir d'un cadre (120), et dans lequel le dispositif modulateur d'écoulement (100) comprend un revêtement (122) sur au moins une partie du composant amont (102) et du composant aval (106), la ou les ouvertures (108) étant définies par une ou plusieurs parties non revêtues du cadre,

et de préférence
dans lequel le cadre (120) définit une pluralité de cellules.

**3.** Dispositif modulateur d'écoulement (100) selon la revendication 2, dans lequel au moins une partie (106d) d'une partie distale du composant aval (106) n'est pas revêtue, la partie distale étant conçue pour s'adapter à la lumière de corps afin d'empêcher ainsi une migration du dispositif modulateur d'écoulement (100) à l'intérieur de la lumière de corps.

4. Dispositif modulateur d'écoulement (100) selon l'une quelconque des revendications précédentes, dans lequel la ou les ouvertures (108) comprennent une pluralité d'ouvertures espacées circonférentiellement autour de la région d'entraînement (104).

5. Dispositif modulateur d'écoulement (100) selon l'une quelconque des revendications précédentes, dans lequel le composant aval (106) comprend une troisième partie divergente (106c) en aval de la seconde partie divergente (106b), l'angle de divergence moyen de la troisième partie divergente étant supérieur à l'angle de divergence moyen de la deuxième partie divergente.

6. Dispositif modulateur d'écoulement (100) selon l'une quelconque des revendications précédentes, dans lequel le dispositif modulateur d'écoulement (100) est configuré pour effectuer une transition entre un état d'administration replié et un état déployé étendu à l'intérieur de la lumière de corps,

et de préférence
dans lequel, à l'état déployé étendu, une partie proximale du composant amont (102) et une partie distale du composant aval (106) sont conçues pour s'adapter à la lumière de corps.

7. Dispositif modulateur d'écoulement (200) selon la revendication 6, dans lequel le composant amont (202) et le composant aval (206) sont formés à partir d'un cadre (210), le dispositif modulateur d'écoulement (200) comprenant un ancrage (230) comprenant :

une partie cadre (232) conçue pour être accouplée au cadre (210) ;
une paire d'entretoises (234) comprenant une partie aval s'étendant à partir de la partie cadre (232) et à distance l'une de l'autre dans une direction amont, et une partie amont s'étendant à partir de la partie aval et l'une vers l'autre dans la direction amont ; et
une partie boucle (236) s'étendant axialement et radialement vers l'extérieur depuis la partie cadre (232) dans la direction amont entre la paire d'entretoises (234), la partie boucle (236) étant conçue pour venir en prise avec la lumière de corps dans l'état déployé étendu pour fixer le dispositif modulateur d'écoulement (200) à l'intérieur de la lumière de corps,
dans lequel, lors de la transition du dispositif modulateur de flux (200) entre l'état déployé étendu et l'état d'administration replié, la paire d'entretoises (234) est conçue pour que les entretoises se déplacent l'une vers l'autre afin d'amener la partie boucle (236) à se contracter radialement vers l'intérieur, de telle sorte que la partie boucle (236) ne s'étend pas radialement au-delà de la paire d'entretoises (234).

8. Dispositif modulateur de flux (100) selon l'une quelconque des revendications précédentes, comprenant en outre une partie récupération (126) s'étendant à partir de l'entrée (101) du composant amont (102) et convergeant dans une direction amont vers une extrémité amont du dispositif modulateur de flux (100), la partie récupération (126) étant conçue pour faciliter la récupération du dispositif modulateur de flux (100),

et de préférence
dans lequel la partie récupération (126) comprend un crochet (130) au niveau de l'extrémité amont du dispositif modulateur de flux (100), le crochet (130) étant conçu pour être tiré afin de replier le composant amont (102).

9. Dispositif modulateur d'écoulement (100) selon l'une quelconque des revendications précédentes, dans lequel le diffuseur décélère l'écoulement de fluide ayant le fluide supplémentaire entraîné passant à travers le composant aval (106).

10. Dispositif modulateur d'écoulement (100) selon l'une quelconque des revendications précédentes, dans lequel la longueur du composant aval est supérieure à la longueur du composant amont.

11. Dispositif modulateur de flux (100) selon l'une quelconque des revendications précédentes, dans lequel l'angle de convergence moyen du composant amont est supérieur à l'angle de divergence moyen du composant aval.

12. Dispositif modulateur d'écoulement (100) selon l'une quelconque des revendications précédentes, dans lequel la ou les ouvertures (108) s'étendent dans la section la plus étroite du dispositif modulateur d'écoulement.

13. Dispositif modulateur d'écoulement (200) selon l'une quelconque des revendications précédentes, comprenant en outre un dispositif de réglage (250, 260) disposé autour d'une circonférence du composant amont (202) à proximité de la sortie (203) du composant amont (202), le dispositif de réglage (250, 260) étant conçu pour être actionné afin de régler un diamètre interne de la sortie (203) in vivo.

14. Dispositif modulateur d'écoulement (200) selon la revendication 13, dans lequel le dispositif de réglage (250) comprend :

un ballonnet annulaire (250) disposé autour de la circonférence du composant amont (202) à proximité de la sortie (203) du composant amont (202) ; et
un orifice d'accès (254) accouplé fluidiquement au ballonnet annulaire (250) par l'intermédiaire d'une conduite de gonflage (252), l'orifice d'accès (254) étant conçu pour recevoir et expulser du fluide pour gonfler et dégonfler ainsi le ballonnet annulaire (250) afin de régler le diamètre interne de la sortie (203) *in vivo.*

15. Dispositif modulateur d'écoulement (200) selon la revendication 13, dans lequel le dispositif de réglage (260) comprend :

une bande (260) disposée autour de la circonférence du composant amont (202) à proximité de la sortie (203) du composant amont (202) ;
un moteur (262) accouplé de manière fonctionnelle à la bande (260) ; et
une unité de commande externe (264) couplée de manière fonctionnelle au moteur (262), l'unité de commande externe (264) étant configurée pour actionner le moteur (262) afin de régler un diamètre de la bande (260) pour régler le diamètre interne de la sortie (203) *in vivo.*

FIG. 1

FIG. 2A

FIG. 2B

FIG. 2C

EP 4 590 235 B1

FIG. 3

FIG. 4A

FIG. 4B

FIG. 5

KIDNEY FUNCTION (GFR) VS. RENAL BLOOD FLOW (RBF)

FIG. 6

FIG. 7

## URINE OUTPUT (ml per 24 hours)

FIG. 8A

## TOTAL NATRIURESIS (mmol per 24 hours)

FIG. 8B

FIG. 9A

FIG. 9B

FIG. 10A

FIG. 10B

FIG. 10C

FIG. 11A

FIG. 11B

FIG. 11C

FIG. 12A

FIG. 12B

FIG. 12C

FIG. 13A

FIG. 13B

FIG. 13C

FIG. 13D

FIG. 13E

FIG. 13F

EP 4 590 235 B1

FIG. 14

230

232

236

234

**FIG. 15A**

200

232 230

236

220

224

234

222

**FIG. 15B**

200

232 234

230

236 222

**FIG. 15C**

FIG. 16A

FIG. 16B

FIG. 17A

FIG. 17B

EP 4 590 235 B1

FIG. 18A

FIG. 18B

FIG. 18C

FIG. 19A

FIG. 19B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63491235
- US 63383884
- WO 2016128983 A
- WO 2018029688 A
- WO 2018220589 A
- WO 2019097424 A
- WO 2020109979 A
- WO IB2022060573 A
- US 10195406 B
- US 11324619 B
- US 20220039938

**Non-patent literature cited in the description**

- **HILLEGE** ; **HANS L. et al.** Renal function, neurohormonal activation, and survival in patients with chronic heart failure. *Circulation*, 2000, vol. 102 (2), 203-210